# FASCICULE DE BREVET EUROPEEN

(11) **EP 1 196 581 B1**
(45) Date de publication et mention de la délivrance du brevet: **29.12.2004**
(21) Numéro de dépôt: 00946004.9
(22) Date de dépôt: 23.06.2000
(51) Int. Cl.: C12N 15/29, C12N 15/82, A01H 5/00

(54) **PROMOTEUR S'EXPRIMANT SPECIFIQUEMENT DANS LES CELLULES DE RACINES DE PLANTES, VECTEURS ET CELLULES HOTES RECOMBINANTES COMPRENANT UN TEL PROMOTEUR ET PLANTES TRANSGENIQUES OBTENUES**
PFLANZENWURZELSPEZIFISCHER PROMOTOR, REKOMBINANTE VEKTOREN UND WIRTSZELLEN DIE DIESEN ENTHALTEN UND KORRESPONDIERENDE TRANSGENE PFLANZEN
PROMOTER EXPRESSED SPECIFICALLY IN THE CELLS OF PLANT ROOTS, RECOMBINANT VECTORS AND HOST CELLS COMPRISING SAME AND TRANSGENIC PLANTS OBTAINED

(30) Priorité: 25.06.1999 FR 9908185
(43) Date de publication de la demande: 17.04.2002
(73) Titulaire: INSTITUT NATIONAL DE LA RECHERCHE AGRONOMIQUE (I.N.R.A.), 75341 Paris Cédéx 07 (FR)
(72) Inventeur: HOFFMANN, Beate, F-78114 Magny les Hameaux (FR); MOLLIER, Pascale, F-91190 Gif sur Yvette (FR); PELLETIER, Georges, F-91440 Bures sur Yvette (FR)
(74) Mandataire: Michelet, Alain
(86) Numéro de dépôt international: PCT/FR2000/001768
(87) Numéro de publication internationale: WO 2001/000833

(56) Documents cités:
- EP-A- 0 824 150
- WO-A-90/07001
- WO-A-91/13992
- WO-A-94/02619
- US-A- 5 110 732
- US-A- 5 837 848
- DATABASE EMBL [en ligne] ACCESSION NO: B26447, 13 octobre 1997 (1997-10-13) ROUNSLEY, D., ET AL.: "F2K20TF IGF Arabidopsis thaliana genomic clone F2K20." XP002133167
- DATABASE EMBL [en ligne] ACCESSION NO: AB025605, 9 avril 1999 (1999-04-09) NAKAMURA, Y.: "Arabidopsis thaliana genomic DNA, chromosome 5, BAC clone:F5H8." XP002151497
- DATABASE EMBL [en ligne] ACCESSION NO: AC012193, 22 octobre 1999 (1999-10-22) LIN, X., ET AL.: "Arabidopsis thaliana chromosome I BAC T32E8 genomic sequence,complete sequence." XP002151480
- DATABASE EMBL [en ligne] ACCESSION NO: AC007289, 13 avril 1999 (1999-04-13) LIN. X., ET AL.: "Arabidopsis thaliana chromosome II BAC F16J10 genomic sequence,complete sequence." XP002133166
- XIANG CHENGBIN ET AL: "DNA-binding properties, genomic organization and expression pattern of TGA6, a new member of the TGA family of bZIP transcription factors in Arabidopsis thaliana." PLANT MOLECULAR BIOLOGY 1997, vol. 34, no. 3, 1997, pages 403-415, XP002133168 ISSN: 0167-4412
- VAN DE RHEE MIRANDA D ET AL: "Analysis of regulatory elements involved in stress-induced and organ-specific expression of tobacco acidic and basic beta-1, 3-glucanase genes." PLANT MOLECULAR BIOLOGY 1993, vol. 21, no. 3, 1993, pages 451-461, XP002133169 ISSN: 0167-4412
- WEI TAO ET AL: "Structure and characterization of a putative drought-inducible H1 histone gene." PLANT MOLECULAR BIOLOGY 1996, vol. 30, no. 2, 1996, pages 255-268, XP002133170 ISSN: 0167-4412
- SUZUKI H ET AL: "Deletion analysis and localization of SbPRP1, a soybean cell wall protein gene, in roots of transgenic tobacco and cowpea." PLANT MOLECULAR BIOLOGY 1993, vol. 21, no. 1, 1993, pages 109-119, XP002133171 ISSN: 0167-4412
- DATABASE BIOSIS [en ligne] BIOSCIENCES INFORMATION SERVICE, PHILADELPHIA, PA, US; juin 1999 (1999-06) KAMO K ET AL: "Tissue specificity and expression level of gusA under rolD, mannopine synthase and translation elongation factor 1 subunit alpha promoters in transgenic Gladiolus plants." Database accession no. PREV199900386225 XP002133172 & PLANT CELL REPORTS JUNE, 1999, vol. 18, no. 10, juin 1999 (1999-06), pages 809-815, ISSN: 0721-7714
- DATABASE BIOSIS [en ligne] BIOSCIENCES INFORMATION SERVICE, PHILADELPHIA, PA, US; 23 novembre 1998 (1998-11-23) OKRESZ LASZLO ET AL: "T-DNA trapping of a cryptic promoter identifies and ortholog of highly conserved SNZ growth arrest response genes in Arabidopsis." Database accession no. PREV199900048625 XP002133173 & PLANT SCIENCE (SHANNON) NOV. 23, 1998, vol. 138, no. 2, 23 novembre 1998 (1998-11-23), pages 217-228, ISSN: 0168-9452
- DATABASE BIOSIS [en ligne] BIOSCIENCES INFORMATION SERVICE, PHILADELPHIA, PA, US; avril 1999 (1999-04) MARTIRANI LUCA ET AL: "T-DNA tagging of nodulation- and root-related genes in Lotus japonicus: Expression patterns and potential for promoter trapping and insertional mutagenesis." Database accession no. PREV199900213703 XP002133174 & MOLECULAR PLANT-MICROBE INTERACTIONS APRIL, 1999, vol. 12, no. 4, avril 1999 (1999-04), pages 275-284, ISSN: 0894-0282
- LINDSEY K ET AL: "TAGGING GENOMIC SEQUENCES THAT DIRECT TRANSGENE EXPRESSION BY ACTIVATION OF A PROMOTER TRAP IN PLANTS" TRANSGENIC RESEARCH,GB,LONDON, vol. 2, no. 1, 1993, pages 33-47, XP002000606 ISSN: 0962-8819

## Description

La présente invention concerne un nouveau promoteur végétal capable de diriger l'expression d'une séquence nucléotidique d'intérêt dans les cellules de la racine d'une plante, ainsi que des vecteurs recombinants contenant un tel promoteur, de préférence associée à une séquence nucléotidique dont l'expression est recherchée dans les cellules constitutives des racines de plantes.

Dans les années récentes, les applications industrielles rendues possibles par les transformations des plantes à l'aide du génie génétique ont été croissantes.

De nombreux gènes d'origine procarycte ou eucaryote (d'animaux ou de plantes), codant spécifiquement pour des protéines conférant de nouvelles propriétés agronomiques, ont été isolés et transférés chez les plantes par génie génétique.

Dans un grand nombre de cas, les gènes qui ont été introduits dans des plantes constituent des séquences chimères, associant des éléments régulateurs d'origines différentes.

Ainsi, le gène codant pour une protéine d'intérêt est souvent placé sous le contrôle d'un promoteur constitutif fort permettant à ladite protéine d'être exprimée dans la totalité de la plante.

A titre d'exemple, le promoteur du transcrit 35S du virus de la mosaïque du chou-fleur (35S CaMV) a été largement utilisé dans des constructions de gènes chimères pour l'expression de protéines d'intérêt chez les plantes.

Désormais, pour un grand nombre d'applications, il n'est pas nécessaire que la protéine d'intérêt conférant la propriété agronomique recherchée présente une expression distribuée dans la totalité des organes et/ou des types cellulaires de la planté transformée.

Très précocement, la recherche d'une expression plus spécifique du gène d'intérêt a été entreprise et a conduit, par exemple, à l'identification de promoteurs spécifiques de tissus ou d'organes.

En particulier, un promoteur dirigeant l'expression d'un polynucléotide d'intérêt de façon à la fois forte et ciblée dans la racine permettrait de nombreuses applications que l'on peut classer comme suit :
(i) défense contre les pathogènes à point d'entrée racinaire, telles que des bactéries, des champignons, des nématodes ou des insectes.
(ii) résistance au stress (froid, stress hydrique, stress salin);
(iii) amélioration de la qualité (exemple: augmenter la teneur en saccharose dans la betterave à sucre);
(iv) nutrition (exemple: exprimer un gène de transporteur des nitrates).

Comme déjà indiqué plus haut, les promoteurs décrits dans l'état de la technique ne permettent pas l'expression d'un polynucléotide d'intérêt dans l'ensemble des couches cellulaires de la racine y compris toutes les assises.

Par exemple, le gène ExtA (WO9113992) est fortement exprimé dans le sclerenchyme racinaire où il participe à l'organisation de la paroi cellulaire, ou encore le gène *arsk1* d'*A. thaliana* (Hwang *et al*., 1995) est exprimé spécifiquement dans la racine, mais son expression est limitée aux couches externes de la racine (épiderme, endoderme, cortex) c'est-à-dire les cellules impliquées dans l'absorption d'eau. L'expression est très faible dans le système vasculaire. Le profil d'expression de ce gène évoque un rôle dans le stress hydrique. De fait, l'expression de ce gène est inductible par un stress hydrique (exposition des racines à l'air, ou traitement des racines par l'ABA ou NaCl), et diminue fortement lorsque les racines sont réhydratées.

Un autre exemple illustratif est le mutant *scarecrow* d'*A*. *thaliana* (Malamy *et al*. 1997) qui est affecté dans l'organisation radiale de la racine: les couches de l'endoderme et du cortex ne s'individualisent pas et restent fusionnées en une couche mutante possédant des caractéristiques de l'endoderme et du cortex. Le gène *scarecrow* affecté par la mutation est exprimé dans l'endoderme, les cellules initiales de l'endoderme, et parfois dans le centre quiescent de la racine.

De plus, les promoteurs décrits dans l'état de la technique, d'une part, ne permettent pas un haut niveau d'expression du polynucléotide d'intérêt, et d'autre part, ne sont pas actifs tout au long du développement de la plante.

Le besoin d'un promoteur végétal fort, spécifique des racines, et actif quel que soit le stade de développement de la plante, est désormais comblé selon la présente invention.

La demanderesse a ainsi isolé, à partir du génome végétal *d'Arabidopsis thaliana*, un nouveau promoteur capable de diriger l'expression d'un polynucléotide d'intérêt spécifiquement dans les racines d'une plante, ledit promoteur assurant un haut niveau d'expression du polynucléotide d'intérêt à la fois dans l'épiderme, le cortex, le vaisseau ou l'endoderme ainsi que dans toutes les assises de la racine, ceci durant tous les stades de développement de la plante.

Ainsi, la présente invention est relative à un acide nucléique isolé caractérisé en ce qu'il comprend un polynucléotide codant pour un promoteur végétal capable de diriger l'expression d'une séquence nucléotidique d'intérêt dans les cellules de la racine d'une plante, durant la totalité du développement de cette dernière, ou un acide nucléique de séquence complémentaire.

De préférence, un acide nucléique selon l'invention se présente sous une forme isolée ou purifiée, à l'exception de la séquence référencée sous le N°AC 007 289 dans la base de données EMBL correspondant à la séquence complète du clone F16J10 map RNS1 du chromosome 2 d'*Arabidopsis thaliana* (Lin X. et al. 1999).

Le terme « isolé » au sens de la présente invention désigne un matériel biologique qui a été soustrait à son environnement originel (l'environnement dans lequel il est localisé naturellement). Par exemple, un polynucléotide présent à l'état naturel dans une plante ou un animal n'est pas isolé. Le même polynucléotide séparé des acides nucléiques adjacents au sein desquels il est naturellement inséré dans le génome de la plante ou l'animal est isolé.

Un tel polynucléotide peut être inclus dans un vecteur et/ou un tel polynucléotide peut être inclus dans une composition et demeurer néanmoins à l'état isolé du fait que le vecteur ou la composition ne constitue pas son environnement naturel.

Le terme « purifié » ne nécessite pas que le matériel soit présent sous une forme de pureté absolue, exclusive de la présence d'autres composés. II s'agit plutôt d'une définition relative.

Un polynucléotide est à l'état purifié après purification du matériel de départ ou du matériel naturel d'au moins un ordre de grandeur, de préférence 2 ou 3 et préférentiellement 4 ou 5 ordres de grandeur.

Aux fins de la présente description, l'expression « séquence nucléotidique » peut être employée pour désigner indifféremment un polynucléotide ou un acide nucléique. L'expression « séquence nucléotidique » englobe le matériel génétique lui-même et n'est donc pas restreinte à l'information concernant sa séquence.

L'invention concerne également un acide nucléique caractérisé en ce qu'il comprend d'un polynucléotide possédant au moins 80% d'identité en nucléotides avec un fragment d'au moins 400 nucléotides consécutifs de la séquence nucléotidique SEQ ID N°1, ou un acide nucléique de séquence complémentaire.

Le « pourcentage d'identité de nucléotides » entre deux séquences, au sens de la présente invention, peut être déterminé en comparant deux séquences alignées de manière optimale, à travers une fenêtre de comparaison. La partie de la séquence nucléotidique dans la fenêtre de comparaison peut ainsi comprendre des additions ou des délétions (par exemple des « gaps») par rapport à la séquence de référence (qui ne comprend pas ces additions ou ces délétions) de manière à obtenir un alignement optimal des deux séquences.

Le pourcentage est calculé en déterminant le nombre de positions auxquelles une base nucléique identique est observée pour les deux séquences comparées, puis en divisant le nombre de positions auxquelles il y a identité des deux bases par le nombre total de positions dans la fenêtre de comparaison, puis en multipliant le résultat par 100 afin d'obtenir le pourcentage d'identité de séquence.

L'alignement optimal des séquences pour la comparaison peut être réalisé de manière informatique à l'aide d'algorithmes connus (par exemple le logiciel FASTA de la Société WISCONSIN GENETICS SOFTWARE PACKAGE, GENETICS COMPUTER GROUP (GCG), 575 Science Doctor, Madison , Wis).

A titre d'illustration, le pourcentage d'identité de séquence pourra être effectué à l'aide du logiciel précité FASTA, en utilisant exclusivement les paramètres par défaut.

Ainsi, les différences nucléotidiques que peut comprendre un acide nucléique selon l'invention par rapport à la séquence nucléotidique SEQ ID N°1 peut résulter en des substitutions, délétions ou additions d'un ou plusieurs nucléotides consécutifs ou non.

Font également partie de l'invention des acides nucléiques comprenant d'un polynucléotide possédant au moins 85%, 90%, 95%, 98%, 99%, 99,5%, ou encore 99,8% d'identité en nucléotides avec la séquence nucléotidique SEQ ID N°1, ou un acide nucléique de séquence complémentaire.

Selon un autre aspect, l'invention est également relative à un acide nucléique caractérisé en ce qu'il comprend d'un polynucléotide hybridant, dans des conditions d'hybridation de forte stringence, avec la séquence nucléotidique SEQ ID N°1, ou un acide nucléique de séquence complémentaire.

Par « partie » d'un polynucléotide promoteur selon l'invention, on entendra une séquence nucléotidique d'une longueur en bases inférieure à celle de la séquence SEQ ID N°1 et conservant la capacité à diriger l'expression d'une séquence nucléotidique d'intérêt dans les cellules de la racine d'une plante.

L'activité biologique d'une partie d'un polynucléotide promoteur selon l'invention peut être aisément vérifiée par l'homme du métier, notamment à l'aide des constructions de vecteurs et des procédés de transformation de plantes avec ces derniers, tels que décrits dans les exemples.

Par « partie » d'un promoteur selon l'invention, on entend notamment les séquences candidates suivantes:
- le polynucléotide allant du nucléotide en position 1 jusqu'au nucléotide en position 2400 de la séquence SEQ ID N°3;
- le polynucléotide allant du nucléotide en position 493 jusqu'au nucléotide en position 2400 de la séquence SEQ ID N°3;
- le polynucléotide allant du nucléotide en position 1076 jusqu'au nucléotide en position 2400 de la séquence SEQ ID N°3;
- le polynucléotide allant du nucléotide en position 1976 jusqu'au nucléotide en position 2400 de la séquence SEQ ID N°3; et
- le polynucléotide allant du nucléotide en position 2040 jusqu'au nucléotide en position 2400 de la séquence SEQ ID N°3.

A titre illustratif, une partie d'un polynucléotide promoteur selon l'invention peut être obtenue par coupure enzymatique d'un acide nucléique tel que décrit ci-dessus, notamment un acide nucléique de séquence SEQ ID N°1, à l'aide d'endonucléases de restriction.

Une « partie » d'un polynucléotide promoteur selon l'invention peut être aussi obtenue par exemple par délétion d'un ou plusieurs nucléotides du polynucléotide de séquence SEQ ID N°1 à l'aide de la technique à l'exonucléase III décrite dans les exemples. Un polynucléotide partie du promoteur végétal selon l'invention a avantageusement une longueur en nucléotides allant de 200, 250, 300, 400, 500, 750, 100, 1200, 1500 ou 2000 nucléotides (ou paire de bases s'il se présente sous la forme double brin).

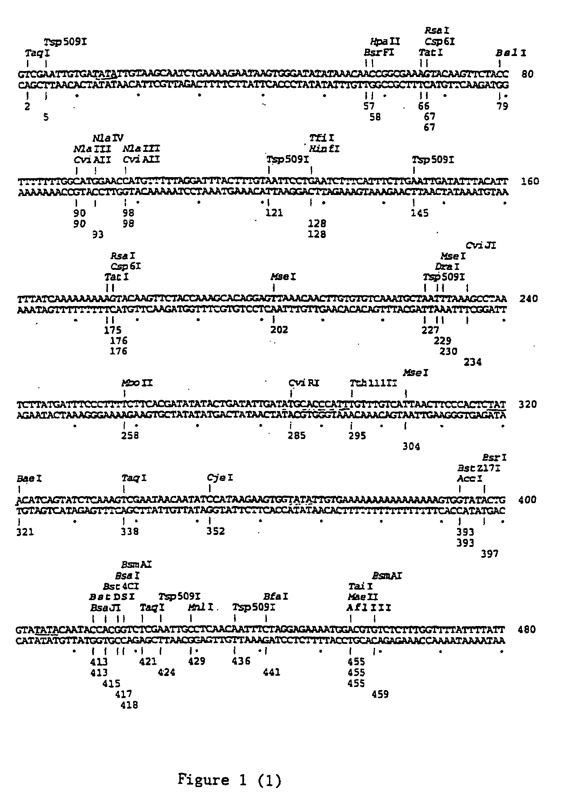

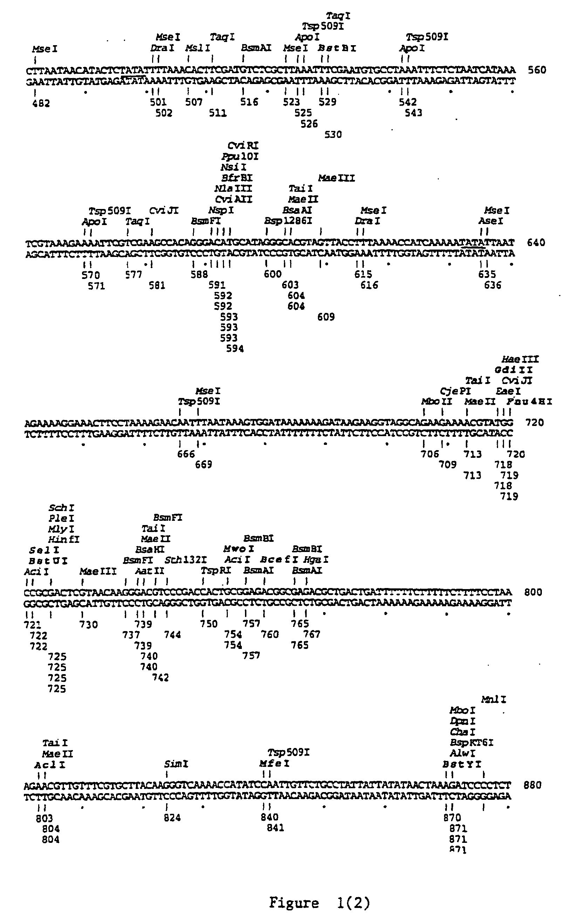

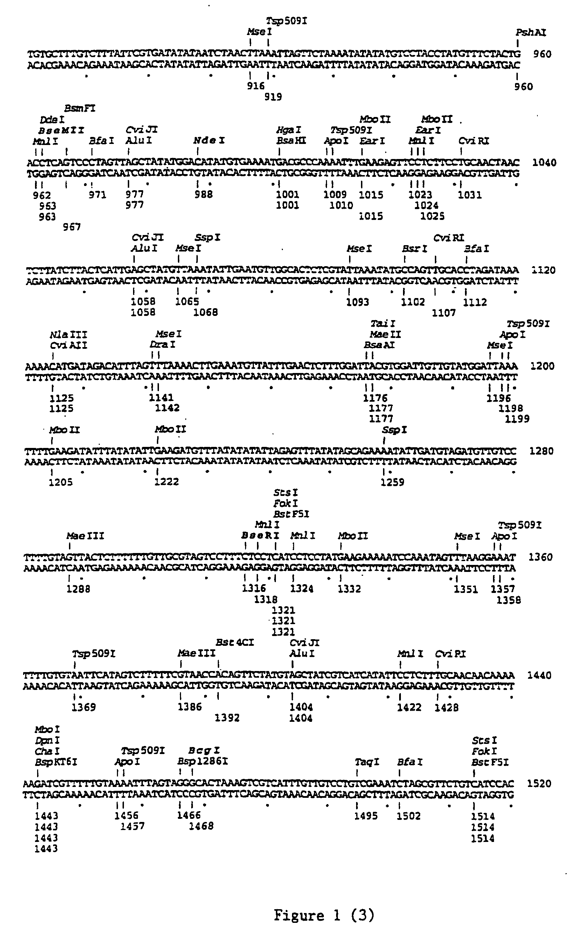

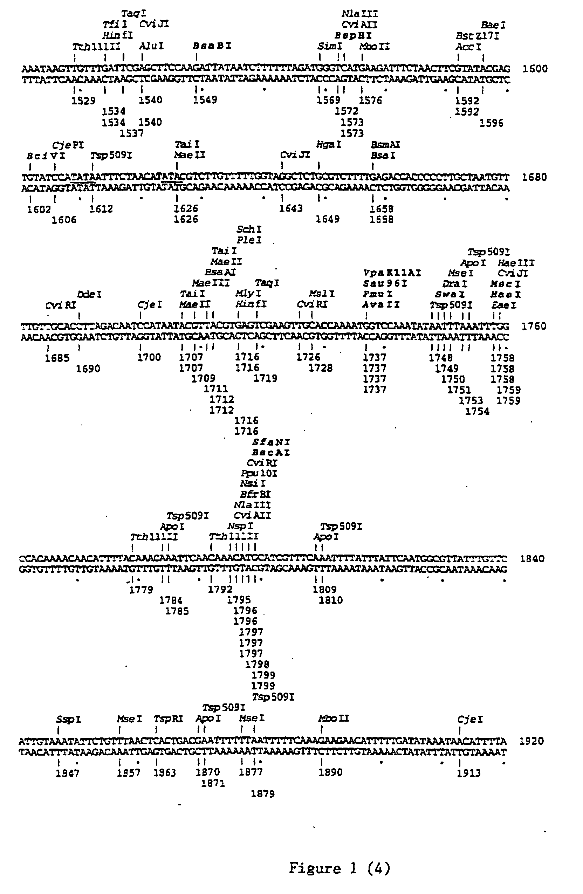

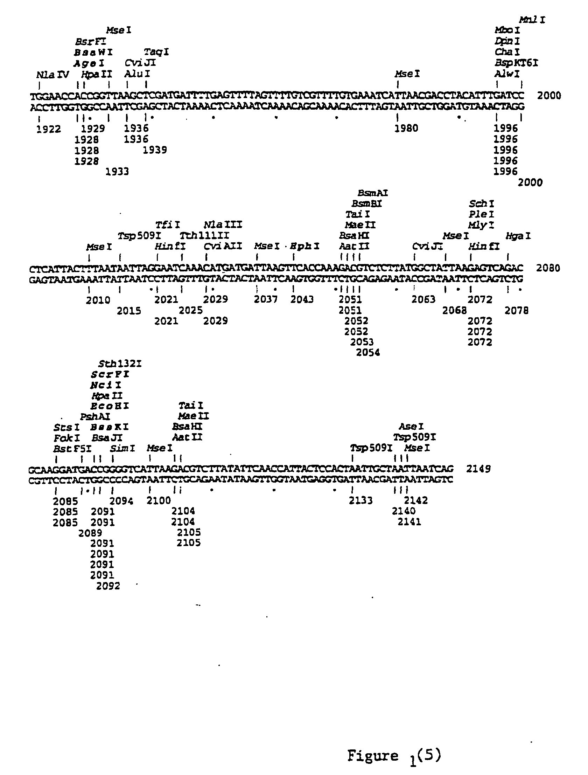

L'homme du métier peut à cet effet utiliser la carte de restriction de la séquence nucléotidique SEQ ID N°1, représentée à la figure 1.

Pour la mise en oeuvre d'enzymes de restriction aux fins d'obtenir des fragments de polynucléotides correspondant à une partie d'un polynucléotide promoteur selon l'invention, l'homme du métier pourra avantageusement se référer à l'ouvrage de Sambrook *et al*. (1989, Molecular Cloning : A Laboratory Manual. 2 ed. Cold Spring Harbor Laboratory, Cold Spring Harbor, New York).

Une partie d'un polynucléotide promoteur selon l'invention pourra également être préparée par amplification spécifique du fragment d'intérêt à l'aide d'un couple d'amorces encadrant, respectivement du côté 5' et du côté 3', la séquence d'intérêt , par exemple à l'aide de la méthode PCR, telle que décrite notamment dans les brevets américains N°US 4 683 195, US 4,683,202 et US 4,965,188.

Par « conditions d'hybridation de forte stringence » au sens de la présente invention, on entendra les conditions d'hybridation suivantes:
- préhybridation des filtres pendant 8 heures à 65°C dans un tampon composé de 6 x SSC, 50 mM Tris-HCl (pH 7.5), 1 mM EDTA, 0,02% PVP, 0,02% Ficoll , 0,02% BSA, et 500 µg par ml d'ADN de sperme de saumon dénaturé;
- hybridation des filtres pendant 48 heures à 65°C en présence de tampon 1 x SSC correspondant à 0,15 M de NaCl et 0,05M de citrate de sodium;
- trois lavages des filtres dans une solution contenant 2 x SSC et 0,1% SDS à 68°C pendant 15 minutes.

Les conditions d'hybridation décrites plus haut sont adaptées à l'hybridation, dans des conditions de forte stringence, d'une molécule d'acide nucléique d'une longueur de 20 nucléotides.

Il va sans dire que les conditions d'hybridation ci-dessus décrites doivent être adaptées en fonction de la longueur de l'acide nucléique dont l'hybridation est recherchée, selon des techniques bien connues de l'homme du métier.

Les conditions convenables d'hybridation peuvent par exemple être adaptées selon l'enseignement contenu dans l'ouvrage de Hames et Higgins (1985, Nucleic Acid Hybridization : A practical approach , Hames and Higgins Ed., IRL Press, Oxford), ou encore dans l'ouvrage de Sambrook *et al*. (1989) précité.

L'invention concerne également un acide nucléique comprenant un polynucléotide promoteur tel que défini ci-avant, caractérisé en ce qu'il comprend en outre une séquence nucléotidique d'intérêt fonctionnellement associée au promoteur végétal et dont l'expression est recherchée dans les cellules de la racine d'une plante.

Un acide nucléique répondant à une telle définition est par exemple l'acide nucléique de séquence nucléotidique SEQ ID N°2 comprenant la séquence du gène *gus* placé sous le contrôle du promoteur de séquence nucléotidique SEQ ID N°1.

De manière avantageuse, un tel acide nucléique comprendra une séquence nucléotidique d'intérêt choisie parmi les séquences codantes de gène interagissant avec des parasites ou des pathogènes tels que les nématodes ou les champignons, comme par exemple les séquences codantes de glucanase, ladite séquence nucléotidique d'intérêt étant placée sous le contrôle d'un polynucléotide promoteur selon l'invention.

Il peut également s'agir de séquences d'endochitinases telles que celles décrites dans le brevet européen n° EP 493,581 ou encore de séquences de gènes agissant sur la teneur en sucre de la plante.

A titre d'exemple, les séquences codantes de gènes d'intérêt assurant la protection d'une plante contre d'autres conditions de stress pourront être avantageusement placées sous le contrôle d'un polynucléotide promoteur selon l'invention.

Stress hydrique ou salin:
- gène arsk1 (Hwang, I. *et al*. ; 1995);
- CDNA pA9 (Winicov, I. , Deutch S.E. 1994);
- CDNA Alfin 1 (Bastola, DR *et al*. 1998).

D'autres séquences codantes pourraient être utilisées sous le contrôle du promoteur selon l'invention, pour agir sur la teneur en saccharose de la betterave à sucre: gène BvSPS1 (Hesse H. *et al*., 1995), ou surexprimer un gène déjà exprimé physiologiquement comme les gènes de transporteurs de nitrate NRT₁ ou NRT₂ ( Crawford, N.M. *et al*. ,1998; Leah R. *et al*., 1991).

Des fragments nucléotidiques comprenant 10 à 2000 nucléotides consécutifs d'un acide nucléique selon l'invention, en particulier d'un acide nucléique possédant au moins 80% d'identité en nucléotide avec la séquence SEQ ID N°1 ou encore un acide nucléique hybridant, dans des conditions d'hybridation de forte stringence, avec la séquence nucléotidique SEQ ID N°1, ou d'un acide nucléique de séquence complémentaire, de tels fragments ayant une longueur de 10, 12, 15, 18 ou 20 à 25, 35, 40, 50, 70, 80, 100, 200, 500, 1000, 1500 ou 2000 nucléotides consécutifs d'un polynucléotide promoteur selon l'invention ou consistant en des fragments d'une longueur de 12, 15, 18, 20, 25, 35, 40, 50, 70, 80, 100, 200, 500, 1000, 1500 ou 2000 nucléotides consécutifs d'un polynucléotide promoteur selon l'invention, pourront avantageusement être mis en oeuvre en tant que sondes ou amorces nucléotidiques aux fins de détection ou d'amplification de la totalité ou d'une partie d'une séquence à activité promoteur spécifique des racines de plantes selon l'invention.

Selon encore un autre aspect, l'invention concerne un vecteur recombinant de clonage et/ou d'expression comprenant un polynucléotide promoteur selon l'invention. Un tel vecteur recombinant comprendra avantageusement une séquence nucléotidique d'intérêt placée sous le contrôle dudit promoteur végétal.

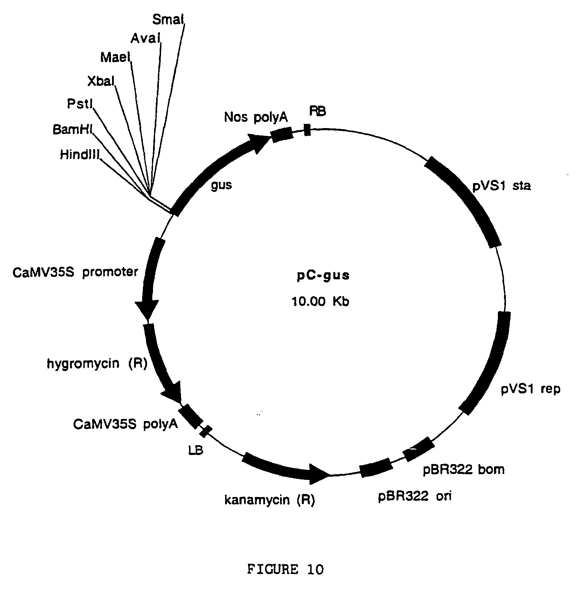

Des vecteurs pouvant être utilisés aux fins de la présente invention sont notamment les suivants:
- vecteur pBIN19 (Bevan *et al*., 1984, Nucleic Acids Research, vol. 12: 8711-8721, commercialisé par la Société CLONTECH , Palo Alto, Californie, USA);
- vecteur 101 (Jefferson , 1987, Plant Molecular Biology Reporter, vol.5: 387-405, commercialisé par la Société CLONTECH);
- vecteur pBI221 (Jefferson, 1987, Plant Molecular Biology Reporter, vol.5: 387-405, commercialisé par la Société CLONTECH);
- vecteur pBI121 (Jefferson, 1987, Plant Molecular Biology Reporter, vol.5: 387-405, commercialisé par la Société CLONTECH).
- vecteur pEGFP (Cormack, B.P. *et al*. 1996; Yang T.T. *et al*., 1996), commercialisé par la Société Clontech.
- vecteur pC-gus représenté à la figure 10.

Un vecteur recombinant préféré selon l'invention est par exemple le vecteur recombinant contenu dans la souche d'*E*. *Coli* déposée à la Collection Nationale de Culture de Micro-Organismes (CNCM) le 25 Mai 1999 sous le n° d'accès I-2218.

L'invention a en outre pour objet une cellule hôte recombinante, caractérisée en ce qu'elle comprend un acide nucléique à activité de promoteur végétal spécifique des racines de plantes selon l'invention, éventuellement associée à un polynucléotide d'intérêt placé sous le contrôle de ce dernier, ou un vecteur recombinant tel que défini ci-dessus.

Les cellules hôtes recombinantes préférées selon l'invention peuvent être indifféremment d'origine bactérienne ou végétale.

Ainsi, peuvent notamment être utilisées des cellules bactériennes de différentes souches de *E. coli* ou encore d'*Agrobacterium tumefaciens*.

Il s'agit également de cellules de plantes transformées par un vecteur conforme à l'invention, tel que des cellules d'*Arabidopsis thaliana*, de colza, de tabac ou encore de maïs.

Une cellule hôte recombinante préférée selon l'invention est la cellule de la souche de *E.coli* déposée à la CNCM le 25 Mai 1999 sous le n°d'accès I-2218.

L'invention concerne aussi un organisme multicellulaire végétal recombinant caractérisé en ce qu'il comprend des cellules hôtes recombinantes telles que définies ci-dessus.

L'invention concerne en particulier une plante transgénique comprenant, sous une forme intégrée dans son génome, un acide nucléique selon l'invention, en particulier un acide nucléique comprenant un polynucléotide promoteur conforme à l'invention et une séquence nucléotidique d'intérêt placée sous le contrôle de ce dernier.

Une plante transgénique selon l'invention peut être notamment un colza, un tabac, un maïs ou encore *Arabidopsis thaliana*.

Les plantes transgéniques telles que définies ci-dessus ont donc la propriété d'exprimer une séquence nucléotidique d'intérêt spécifiquement au niveau des différents types cellulaires de la racine ( de l'extérieur vers l'intérieur: épiderme, cortex, endoderme, pericycle, vaisseau ), à tous les stades de développement de la plante.

L'invention a en outre pour objet un procédé d'obtention d'une plante transgénique exprimant spécifiquement une séquence nucléotidique d'intérêt dans les cellules de la racine à tous les stades de développement de ladite plante, caractérisé en ce qu'il comprend les étapes suivantes:
a) obtention d'une cellule hôte recombinante végétale conforme à l'invention;
b) régénération d'une plante entière à partir de la cellule hôte recombinante obtenue à l'étape a);
c) sélection des plantes obtenues à l'étape b) ayant intégré la séquence nucléotidique d'intérêt placée sous le contrôle du polynucléotide promoteur végétal selon l'invention.

L'invention est également relative à un procédé d'obtention d'une plante transgénique caractérisé en ce qu'il comprend les étapes suivantes:
a) obtention d'une cellule hôte recombinante d'*Agrobacterium tumefaciens* contenant une séquence nucléotidique d'intérêt placée sous le contrôle du polynucléotide promoteur végétal selon l'invention;
b) transformation de la plante d'intérêt par infection avec la cellule hôte recombinante d'*Agrobacterium tumefaciens* obtenue à l'étape a);
c) sélection des plantes ayant intégré la séquence nucléotidique d'intérêt placée sous le contrôle du polynucléotide promoteur végétal selon l'invention.

L'invention a en outre pour objet un procédé d'obtention d'une plante transgénique caractérisé en ce qu'il comporte les étapes suivantes:
a) transfection d'une cellule de plante avec un acide nucléique ou un vecteur recombinant comprenant une séquence nucléotidique d'intérêt placée sous le contrôle du polynucléotide promoteur selon l'invention;
b) régénération d'une plante entière à partir de cellules de plante recombinantes obtenues à l'étape a) ;
c) sélection des plantes ayant intégré la séquence nucléotidique d'intérêt placée sous le contrôle du polynucléotide promoteur végétal selon l'invention.

L'un quelconque des procédés d'obtention d'une plante transgénique ci-dessus décrit peut en outre comporter les étapes additionnelles suivantes:
d) croisement entre elles de deux plantes transgéniques telles qu'obtenues à l'étape c);
e) sélection des plantes homozygotes pour le transgène.

Selon une autre alternative, l'un quelconque des procédés ci-dessus décrits pourra en outre comprendre les étapes suivantes:
d) croisement d'une plante transgénique obtenue à l'étape c) de l'un quelconque de ces procédés avec une plante de la même espèce;
e) sélection des plantes issues du croisement de l'étape d) ayant conservé le transgène.

L'invention a en outre pour objet une plante transgénique telle qu'obtenue selon l'un quelconque des procédés ci-dessus.

De manière préférée, une plante transgénique selon l'invention a non seulement intégré dans son génome un transgène comprenant une séquence nucléotidique d'intérêt placée sous le contrôle du polynucléotide promoteur végétal présentement décrit mais exprime ladite séquence nucléotidique d'intérêt majoritairement ou exclusivement dans les cellules constitutives de la racine.

Enfin, l'invention concerne aussi une semence de plante dont les cellules constitutives comprennent dans leur génome un acide nucléique selon l'invention.

Il s'agit notamment d'une semence de *Arabidopsis thaliana*, de colza, de tabac ou de maïs ayant incorporé un acide nucléique selon l'invention.

L'invention sera en outre illustrée, sans pour autant être limitée, par les figures et les exemples suivants.

La figure **1** représente une carte de restriction de la séquence nucléotidique SEQ ID N°1.

Les motifs suivants ont été identifiés dans cette séquence: deux motifs TGACG correspondant au site de fixation du facteur Asfl spécifique de la racine dans le promoteur 35 S du CaMV (position 1000-1004 et 1866-1870), deux motifs proches, à un nucléotide près, de séquences enhancer du même promoteur 35S (position 28-35: CTGAAAG, au lieu de GTGAAAG et position 882-889: GTGCTTTG, au lieu de GTGGTTTG) et 3G-box ACGT (positions 285-288, 604-607, 1107-1110). Par ailleurs, cette séquence comporte 21 motifs TATA et 9 motifs CAAT.

L'importance fonctionnelle de ces motifs pourra être évaluée par la méthode à l'exonucléase III, selon Ausubel *et al*. (1989). Cette méthode permet d'obtenir des fragments de promoteur de taille décroissante, qui seront clonés en amont du gène *gus* dans un vecteur permettant la transformation d'*Arabidopsis*.

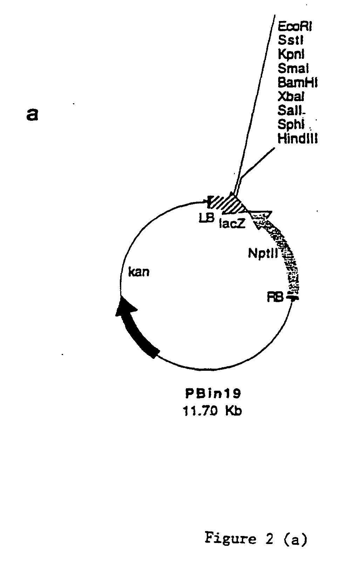

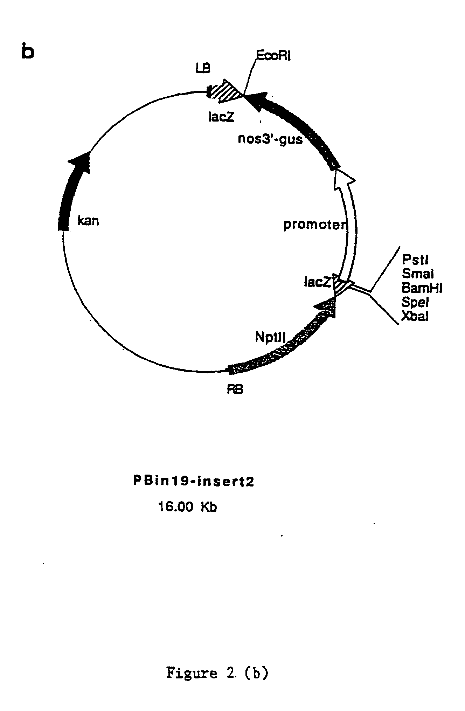

La figure **2** illustre la construction 1 ayant été utilisée pour l'isolement du promoteur selon l'invention, en l'absence (figure 2a) ou en présence (fig.2b) de l'insert.

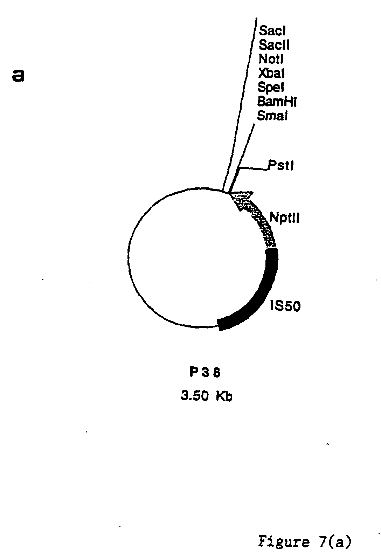

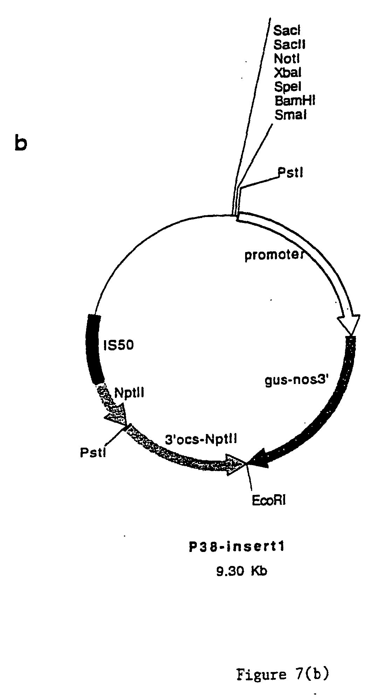

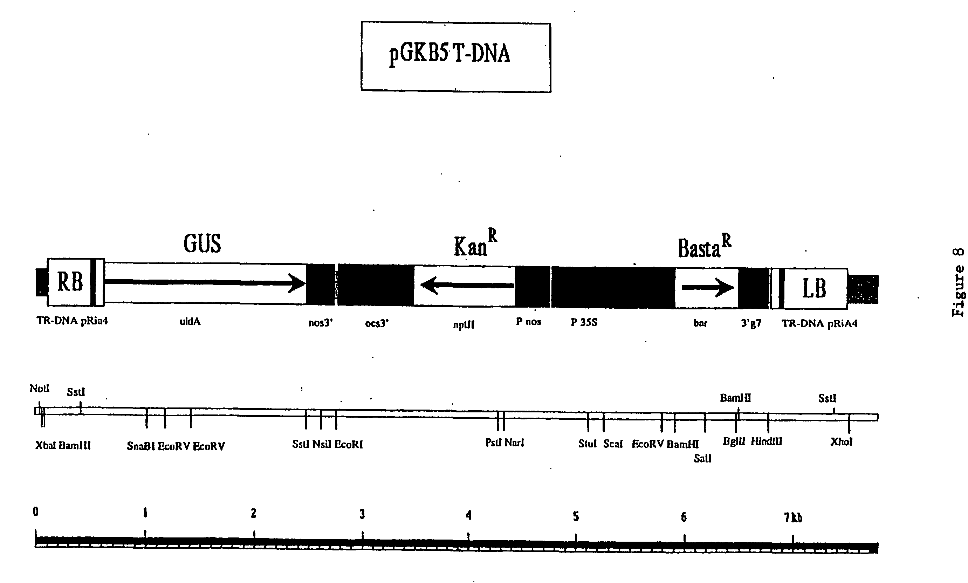

L'insert de 4.27 kb est cloné à partir du vecteur de « kanamycin rescue » (figure 7) dans l'ADN-T du vecteur pBin19 par une double digestion EcoRI-XbaI. Cet insert comporte 2.14 kb de séquence génomique du clone Ir1 ( SEQ ID N°3 nct 136-2284) et 2.13kb de l'ADN-T de pGKB5: séquence codante de *gus* et signal de polyadénylation nos (figure 8 - nct 632-2762).
LB : bordure gauche de l'ADN-T de pBin19.
LacZ: lacZ région du phage M13mp19.
NPTII: fragment contenant le promoteur nos, le gène de résistance à la néomycine, et le site de polyadénylation nos.
RB : bordure droite de l'ADN-T de pBin19.
kan: fragment contenant l'origine de réplication RK2 du plasmide pRK252 et le gène kan de résistance à la kanamycine de *Streptococcus*.

La figure **3** illustre l'expression GUS du transformant d'*Arabidopsis* (écotype WS) au cours du développement.
a- 7 jours après germination.
b- 14 jours après germination.
c- 24 jours après germination.
d- détail d'une racine.

La figure **4** illustre une coupe transversale de racine du transformant après révélation de l'activité GUS.

La figure **5** réprésente une autoradiographie d'un Northern blot hybridé avec une sonde GUS.

6 µg d'ARN ont été déposés dans chaque puits.
Puits n°1-3-5: ARN de parties aériennes du transformant homozygote n°1, n°13 et de plante non transformée WS, respectivement.
Puits n°2-4-6: ARN de racines des mêmes plantes.

La figure **6** illustre l'analyse quantitative de l'expression GUS de transformants d'*Arabidopsis* obtenus avec la construction 1, au cours du développement. Elle matérialise la comparaison de l'activité GUS dans les racines et la partie aérienne du transformant initial (a) et des transformants individuels caractéristiques 6-1 et 2b (b-c), au cours du développement.

L'activité gus est exprimée en unités de fluorescence par minute et par :
- 1 µg de protéines (racines)
- 20 µg de protéines (feuilles)

A 2,72 unités de fluorescence correspond 1 pmol du produit mu, qui est le produit de la catalyse enzymatique du substrat mug (4-méthyt-β-D glucuronide) par GUS.

La figure **7** illustre le vecteur obtenu à la suite du « Kanamycin rescue ». La technique de « kanamycine rescue » utilise le vecteur P38 (a), qui comporte le début du gène NptII de résistance à la kanamycine, jusqu'au site Pstl, en aval d'un promoteur IS50. Après digestion par PstI du vecteur P38 et de l'ADN du transformant, ligation des deux et sélection sur kanamycine, on obtient le vecteur montré en b). L'insert 1 est le fragment PstI obtenu à partir de l'ADN génomique du transformant: il comporte la région promotrice (SEQ ID N°1, nct 1 à 2149) accolée au fragment d'ADN-T délimité par le site d'insertion côté RB et le site PstI situé dans le gène de la kanamycine (figure 8, nct 632 à 4279). Le gène de résistance à la kanamycine se trouve ainsi reconstitué et le vecteur recombinant est sélectionné sur kanamycine.

La figure **8** donne une représentation schématique de l'ADN-T de PGKB5 utilisé pour créer la collection de transformants de Versailles.

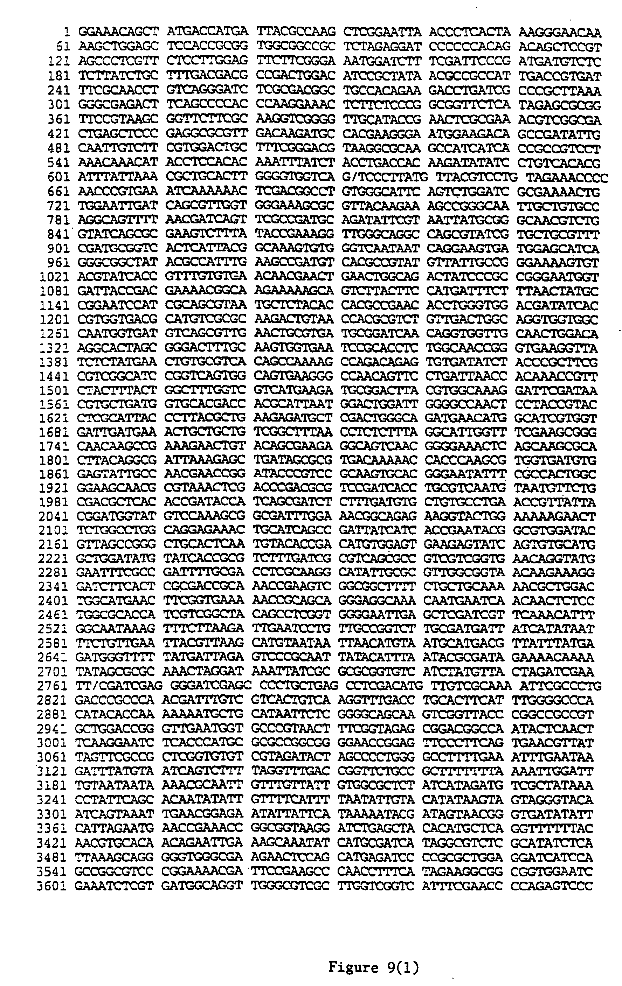

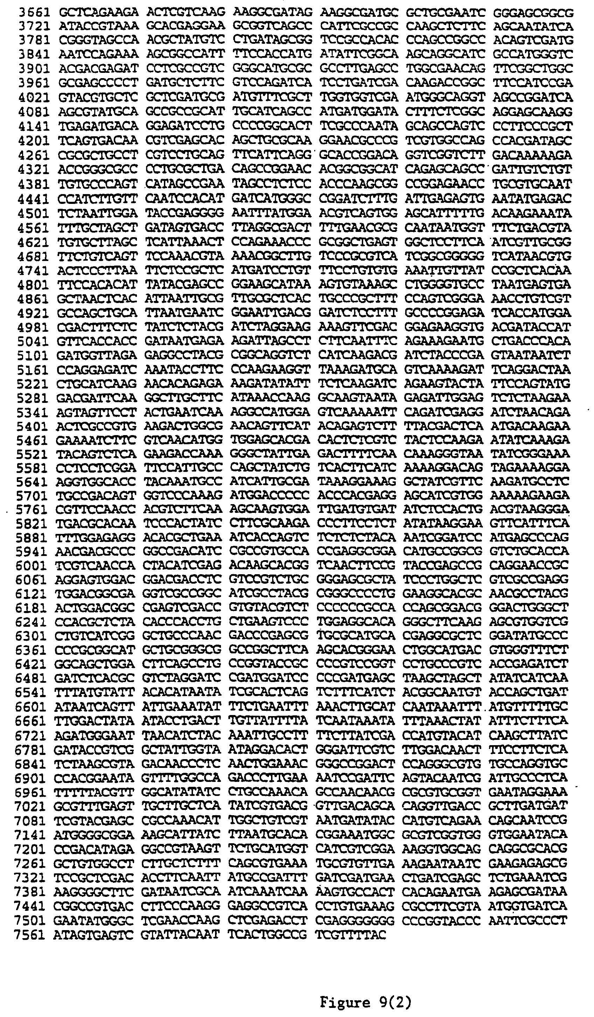

La figure **9** illustre la séquence de l'ADN-T de PGKB5, également référencée comme la séquence SEQ ID N°5.
- RB bordure de 24 pb: 574-596,
- gène *gus* : séquence *gus* sans promoteur : 638-2504 (ATG:638-640, codon stop: 2444-2446), site de polyadénylation du *gus*: 3'nos: 2505-2793, site EcoRI: AATT/C:2759-2763.
- gène *KanR* : promoteur nos: 4752-4480, séquence *KanaR:* 4479-3490 (ATG: 4466-4464, codon stop : 3665-3663, site Pstl: CTGCA/G : 4275-4280), site ocs3': 3489-2794.
- gène *PhosphinothricineR* (bastaR): promoteur 35S: 4767-5890, séquence *phosphinotricineR*: 5890-6503 (ATG:5930-5932, codon stop: 6480-6482), site 3'g7: 6504-6789.
- LB bordure de 24 pb: 6962-6986.

La figure **10** représente une carte détaillée du vecteur pC-gus utilisé dans l'exemple 5.

### EXEMPLES:

### MATERIELS ET METHODES:

### I - Transformation

(Bechtold N., Ellis J., Pelletier G. , 1993. *Agrobacterium* mediated gene transfer by infiltration of adult *Arabidopsis thaliana* plants. *C.R. Acad. Sci. Paris* **316**: 1194-1199.)

6 mg de graines (soit environ 300 graines) d'*Arabidopsis thaliana* d'écotype Wassilevskija ont été semés sur des bacs de compost 40 x 30 cm. Les bacs sont laissés 64 h à 4°C pour la germination, puis sont placés en serre (photopériode : 16h de jour, température : 15° C nuit / 25°C minimum jour) et arrosés avec la solution nutritive standard de Coïc et Lessaint (Coïc, Y., Lessaint, C. 1971. Comment assurer une bonne nutrition en eau et ions minéraux en horticulture. *Hortic. fr.*8:11-14).

MP5-1 *Agrobacterium* est cultivé dans du milieu LB ( Luria-Bertani, Sambrook, J., Fritsch, E. F., Maniatis,T. 1989. Molecular Cloning : A Laboratory Manual. Cold Spring Harbor, New York ) avec 50 mg/l de rifampicine, 100 mg/l de gentamycine et 200 mg/l de kanamycine, 14h à 28°C (jusqu'à A600=0.8). Après centrifugation, le culot de bactéries est remis en suspension dans le milieu d'infiltration (MI), à un tiers du volume de culture initial (MI= macro et micro nutriments de Murashige et Skoog, contenant 10 µg/l 6-benzylaminopurine et 5% sucrose (Murashige, T., Skoog, F. 1962. A revised medium for rapid growth and bioassays with tobacco tissue cultures. *Physiol Plant 15* : 473-497). Des lots de 100 à 500 plantes de 3 à 4 semaines bien développées ont été extraits du sol, rincés à l'eau, immergés dans 2I de milieu MI contenant *Agrobacterium* dans une cloche à vide de 10l de contenance. Les plantes sont maintenues sous vide (10⁴ Pa) pendant 20 mn. Toutes les manipulations des plantes traitées, jusqu'à leur récolte, ont été effectuées avec des gants en latex. Les plantes traitées ont été plantées dans un nouveau compost, à raison de 54 plantes par bac, puis incubées 2 jours sous un plastique pour prévenir toute déshydratation et pour faciliter l'enracinement. Quatre à six semaines après la plantation, la génération T1 a été récoltée en mélange. Les plants ont été sélectionnés sur du sable irrigué avec de l'eau contenant de l'herbicide Basta (5-10 mg/ml phosphinothricine). Deux mois plus tard, les graines T2 ont été récoltées individuellement et conservées pour les analyses ultérieures.

### II - « Kanamycin rescue »

(Bouchez D., Vittorioso P., Courtial B., Camilleri C., 1996. Kanamycin Rescue: A Simple technique for the recovery of T-DNA flanking sequences. *Plant Mol. Biol. Rep.* **14**: 115-123.)

### - Extraction de l'ADN génomique

De 0,5 à 0,75g de feuilles sont congelés rapidement dans l'azote liquide, broyés, en présence de polyclar™, en fine poudre dans un mortier à l'aide d'un pilon, et la poudre est transférée avec l'azote liquide dans un tube "Oak Ridge", dans lequel sont ajoutés 15ml de tampon d'extraction (Tris 100mM, EDTA 50mM, NaC I500mM, β-mercaptoéthanol 10mM, pH8). Après addition de 1ml de SDS 20%, les tubes sont incubés à 65°C pendant 10mn en agitant toutes les 3 à 4mn. 5ml d'acétate de potassium (5M) sont ajoutés et incubés à 0°C pendant au moins 20mn. Après centrifugation à 25000g (13000rpm) pendant 20mn, le surnageant est filtré à travers un filtre Miracloth (Calbiochem) dans un tube de 30ml contenant 10ml d'isopropanol et incubé à -20°C pendant 30mn. Après centrifugation à 20000g (10000 tpm) pendant 15mn, le culot d'ADN est séché en renversant le tube sur du papier absorbant pendant 10mn. L'ADN est repris dans 0,7ml de TE 50/10 (Tris 50mM, EDTA 10mM pH8) auxquels sont ajoutés 5 µl de RNAse (5mg/ml) et incubé à 37°C pendant 10mn. L'ADN est extrait par un volume égal de phénol/chloroforme 1/1 et précipité à l'isopropanol (1volume)/ NaOAc (3M) (1/10 de volume). Le culot d'ADN est séché et repris dans 10µl de TE 10/1 (Tris 10mM, EDTA 1 mM, pH8).

### - Clonage

*Première digestion*. 0.5 µg d'ADN génomique d'*Arabidopsis* sont digérés avec PstI (BRL life technologies, 95613, Cergy-Pontoise), précipités à l'ethanol (2.5 volumes)/ NaOAc 3M (1/10 du volume) et remis en suspension dans de l'eau. 2.5 µg du vecteur pResc38 sont digérés par PstI, déphosphorylés avec de la phosphatase alcaline d'intestin de veau (BRL), extraits avec un volume de phénol-chlorophorme (1/1) précipités à l'éthanol/ NaOAc, et remis en suspension dans de l'eau.

*Première ligation*. 0.5 µg d'ADN génomique digéré par Pstl et 2.5 µg de pResc38 digéré par Pstl et déphosphorylé sont mis à liguer dans 100 µl de volume total avec 5 unités de T4 ADN ligase (BRL), toute la nuit à 12°C.

*Deuxième digestion*. Le mélange de ligation précédent est précipité à l'éthanol (2.5 volumes)/ NH4OAc 8M (1/2 du volume), remis en suspension dans de l'eau, et complètement digéré avec un deuxième enzyme de restriction : Xbal, dans un volume total de 100 µl en utilisant 20 unités d'enzyme de restriction. Le mélange est précipité à l'éthanol/NH4OAc et mis en suspension dans de l'eau.

*Deuxième ligation*. Afin de circulariser les molécules d'ADN, une deuxième ligation est réalisée sur le produit de la deuxième digestion avec une concentration d'ADN plus faible, dans un volume total de 200 µl et en utilisant 5 unités de T4 ADN ligase. Le mélange est incubé toute la nuit à 12°C, puis précipité à l'éthanol/ NH4OAc, rincé 2 fois à l'éthanol 70% (v/v), séché, et repris dans 20 µl d'eau.

### - Transformation

L'électroporation est réalisée grace à un appareil de type Gene-Pulser (Bio-Rad Laboratories, Richmond, CA) avec un voltage de 1,5 kV. Les cellules electrocompétentes DH10B electromax (BRL) sont rapidement décongelées puis placées sur la glace. Dans une cuvette d'electroporation froide (diamètre interélectrode de 1mm, Bio-Rad), sont mélangés 2 µl du produit de ligation précipité et 40 µl de cellules compétentes. Après électroporation, 1 ml de milieu SOC froid (Sambrook, J., Fritsch, E. F., Maniatis,T. 1989. Molecular Cloning : A Laboratory Manual. Cold Spring Harbor, New York) est ajouté immédiatement. L'ensemble est transvasé dans un tube de culture de 13 ml et incubé 2h à 37°C sous agitation.

Un volume de 250 µl de culture est étalé sur des boîtes de Pétri LB-agar contenant 100 mg/l de carbenicilline et 50 mg/l de kanamycine et incubé à 37°C toute la nuit.

### III - Clonage dans Pbin19

L'insert cloné dans le vecteur P38resc de "Kanamycin rescue" subit un clonage intermédiaire dans le vecteur Bluescript pBKS+ (Stratagène, San Diego CA92121) avant d'être cloné dans le vecteur binaire pBin19 en vue de la transformation des plantes. Ces clonages se font de manière directionnelle par double digestion EcoRI/XbaI.

Environ 250 ng de vecteur P38resc contenant l'insert et digéré par EcoRI et Xbal sont ligués avec environ 100 ng de vecteur KS+ digéré par les mêmes enzymes, non déphosphorylé, dans 40 µl de volume final avec 10 U de T4 ADN ligase (BRL). Après incubation une nuit à 12 °C, le mélange de ligation est précipité à l'éthanol/ NH4OAc, repris dans 10 µl d'eau et utilisé pour électroporer des bactéries NM522 (BRL), rendues électrocompétentes suivant la technique décrite par Sambrook *et al*.,(1989). Les colonies positives blanches sont sélectionnées sur un milieu LB-agar contenant 40 mg/l de XGaI, 8 mg/l d'IPTG (Genaxis Biotechnology, 78180, Montigny le Bretonneux) et 100mg/l de carbénicilline.

### -Clonage dans pBin19.

L'insert contenu dans pBKS+, après digestion par EcoRI et Xbal, est purifié par électroélution à partir d'un gel d'agarose à 1%, selon la technique de Sambrook *et al*., (1989). Pour le clonage, 100ng de l'insert de 4.3 kb et 100ng de vecteur pBin19 (12 kb) préalablement digéré par EcoRI et Xbal (soit un rapport molaire insert/vecteur de 3/1) sont mélangés dans 40 µl de volume total avec 10 µl de ligase (BRL) et ligués sur la nuit à 12 °C. Après précipitation à l'éthanol/ NaOAc, le produit de ligation est repris dans 10 µl d'eau et utilisé pour réaliser l'électroporation des bactéries NM522. La sélection des colonies positives se fait sur des boîtes de Pétri avec un milieu LB-agar contenant XGaI et IPTG, comme ci-dessus et 50mg/l de kanamycine.

### IV - Méthode à l'exonucléase III

(*Current Protocols in Molecular Biology*, éditeurs: F.M. Ausubel, R. Brent, R.E. Kingston, D.D. Moore, J.G. Seidman, J.A. Smith, K. Struhl; published by Wiley Interscience)

Le fragment d'ADN d'interêt de 4,3kb a été recloné dans un vecteur pBluescript II KS+ au site EcoRI du polylinker. Ensuite le plasmide de ce clone a été isolé et purifié par la méthode de "Qiagen-Midi-Preparation Tip 100( Qiagen) à partir d'une culture de 30 ml.

Pour pouvoir séquencer dans les deux sens, 5µg plasmide ont été doublement digérés par XhoI/KpnI d'une part et par Spel/Sacl d'autre part, dans un volume de 50 µl chaque fois. (100ng de plasmide linéarisé de chaque digestion a été gardé pour une vérification sur gel d'agarose). Le reste du plasmide linéarisé à chaque digestion a été précipité à l'éthanol 95% (3 volumes) et NaOAc 3M (1/5 volume) pendant une heure dans la glace. Après 20 minutes de centrifugation à 13000 tpm et à +4°C, le plasmide digéré de chaque digestion a été rincé avec l'éthanol à 70%, seché au "speed-vac" pendant 5 minutes et repris dans 50µl de tampon ExoIII dilué à 1x (0,66M Tris/HCl pH=8,0 , 66 mM MgCl₂, 50mM DTT, 500µg /ml BSA; USB, United States Biochemicals).

Pour créer les délétions de chaque côté, 25µl ( 2,5µg ) de chaque digestion ont été préincubés à 37°C pendant 2 minutes, 0,8µl de ExoIII (100u/µl; USB), soit 150unités ExoIII par picomole d'extrémités 3', ont été ajoutés et reincubés à 37°C. Toutes les minutes, 3µl (300ng) d'ADN ont été prélévés et placés immédiatement dans la carboglace (=8 prélèvements totaux). Puis 3µl d'eau ont été ajoutés à chaque prélèvement , incubés pendant 10 minutes à 70°C pour inactiver l'enzyme ExoIII. Tous les échantillons ont été placés dans la glace. Après addition de 15 µl de tampon de nucléase S1 (300mM Na acétate pH 4,6 ,10mM Zn acétate, 50% v/v glycérol) et de 4µl (4 unités) de nucléase S1 (Gibco BRL) , ces échantillons ont été incubés pendant 20 minutes à température ambiante. La réaction de la nucléase S1 a été arrêtée en ajoutant 5µl de tampon "stop" (0,3M Tris/HCl pH 8,0 , 0,05M EDTA) à chaque échantillon . Des fractions aliquotes de 8µl ont été prélévées pour une vérification sur gel d'agarose.

Le volume restant (22µl) de chaque échantillon a été incubé pendant 20 minutes à 37°C après avoir ajouté 2 unités de fragment de Klenow et 1µl de dNTP's à 0,25mM.

Enfin les molécules délétées ont été recircularisées en ajoutant 1µl (1 unité) de T₄DNA ligase (USB), 3µl de tampon 10x (660mM Tris/HCl pH=7,6, 66mM MgCl₂, 100mM DTT, 660µm ATP) et 2µl d'eau à chaque échantillon. Les ligations ont été faites dans un volume total de 30 µl et incubées à 16 °C pendant la nuit.

Puis un tiers du volume (10µl) des produits issus de chaque ligation a été utilisé pour transformer 100µl de cellules compétentes *E.coli* DH5α par la méthode au Chlorure de Calcium ( Current Protocols in Molecular Biology, éditeurs: F.M. Ausubel, R. Brent, R.E. Kingston, D.D. Moore, J.G. Seidman, J.A. Smith, K. Struhl,; published by Wiley Interscience ) . Les cellules transformées de *E. coli* DH5α ont été selectionnées sur LB-agar contenant de la carbénicilline à 100mg/l , 40 mg/l de XGaI et 8 mg/l d'IPTG.

### V - Extraction des ARN totaux

(Heim U., Weber H., Bäumlein H., Wobus U., 1993. A sucrose-synthase gene of *Vicia faba L*.: expression pattern in developing seeds in relation to starch synthesis and metabolic regulation. *Planta* **191**: 3494-501)

Les tissus frais congelés (plantule ∼2g et racine ~1g) ont été écrasés dans l'azote liquide à l'aide d'un mortier et un pilon. Puis 500µl de tampon d'extraction (1M Tris/HCl pH 7,4 , 1% SDS , 5mM EDTA) pour 200mg tissu ont été ajoutés goutte à goutte, puis le même volume de phenol/chloroformelisoamylalcohol, tout en continuant le broyage jusqu'a obtenir une poudre lisse. Après décongélation, chaque solution a été transferée dans un tube et centrifugée pendant 5 minutes à +4°C .

Chaque phase aqueuse a été réextraite deux fois avec le même volume de phénol/chloroforme/isoamylalcohol et précipitée à l'éthanol (3 volumes) / NaOAC ( 1/10 volume) pendant une heure à -80°C. Après centrifugation pendant 30 minutes à +4°C, chaque culot a été séché brièvement et dissous dans de l'eau+DEPC . Une deuxième centrifugation pendant 10 minutes à +4°C a été faite et chaque surnageant a été mélangé avec le même volume de LiCl à 4M pour une précipitation des acides ribonucléiques dans la glace à +4°C pendant la nuit.

Ensuite chaque solution a été centrifugée pendant 15 minutes et les culots d'ARN ont été lavés deux fois dans LiCl 2M et une fois dans de l'éthanol à 70% . Apès un séchage au "speed-vac , chaque culot d'ARN a été dissous dans de l'eau+DEPC et la concentration d'ARN a été vérifiée par spectrophotométrie.

### VI - Test GUS

(Jefferson R. A., 1987. Assaying chimeric genes in plants: the *gus* gene fusion system. *Plant Mol. Biol. Rep*. **5**: 387.)

### - Par histochimie

Quinze jours après la germination des transformants d'*Arabidopsis*, l'activité GUS est téstée grâce à l'acide X-glucuronique (X-Glu, Biosynth G. Staad, Suisse) comme décrit par Jefferson *et al*., modifié par l'utilisation de 100 mM KH2PO₄, 0.4 mM de catalyseur K3Fe(CN)6 et 0.4 mM K4Fe(CN)6. Aucun bruit de fond n'a été observé dans les tissus des plantes non transformées.

### - Par fluorimétrie

Les échantillons de plantes (racines ou feuilles) sont broyés en tube Eppendorf™ avec 200 µl de tampon d'extraction ( 50mM NaPO₄, 10 mM dithiothreitol, 10 mM EDTA, pH7) et une pincée de sable de Fontainebleau. Après centrifugation deux fois 10 min à 13000tpm à 4°C, le dosage d'activité GUS est réalisé sur le surnageant, dans 150µl de volume final contenant le substrat MUG (4-Methyl-β-D-glucuronide d'ombellifère, Sigma) à concentration finale de 3 mM.

Après 15 min d'incubation à 37°C, l'activité GUS est mesurée grâce à un appareil Fluoroskan Il ( Labsytems, 91944 Les Ulis, France) avec des longueurs d'onde d'excitation et d'émission de 365 nm et 455 nm, respectivement.

Les concentrations de protéines dans les extraits de plantes sont mesurées en utilisant le réactif de Bradford (Biorad).

Les concentrations d'ADN sont mesurées en utilisant le réactif de Hoechst (Sigma). La réaction se fait dans 200 µl de volume final (tampon Labarca-Paigen : 50 mM NaPO₄, NaCl 2M, EDTA 2mM, pH 7.5) contenant le réactif de Hoechst à 0.5 mg/ml.

### EXEMPLE 1: Isolement d'une séquence nucléotidique d'environ 2,2 kb par piégeage de promoteur.

Une collection de transformants d'*Arabidopsis thaliana* (écotype WS) a été obtenue selon la technique décrite par Bechtold *et al*. (1993).

Les plantes ont été transformées par insertion aléatoire dans leur génome d'un ADN de transfert (ADN-T) transmis par la bactérie *Agrobacterium tumefaciens*.

Cet ADN de transfert comporte un gène *gus* sans promoteur tel que décrit par Bouchez *et al*. (1993, C.R.A.S. Paris, volume 316:1188-1193).

La méthode de transformation *In planta* a été choisie et mise au point à la Station Génétique de Versailles de l'Institut National de la Recherche Agronomique selon la méthode décrite par Bechtold *et al*. (1993, C.R.A.S. Paris, volume 316:1194-1199). Cette méthode permet d'obtenir rapidement un nombre élevé de transformants indépendants comportant un nombre limité d'insertions (1,5 insertions par transformant en moyenne).

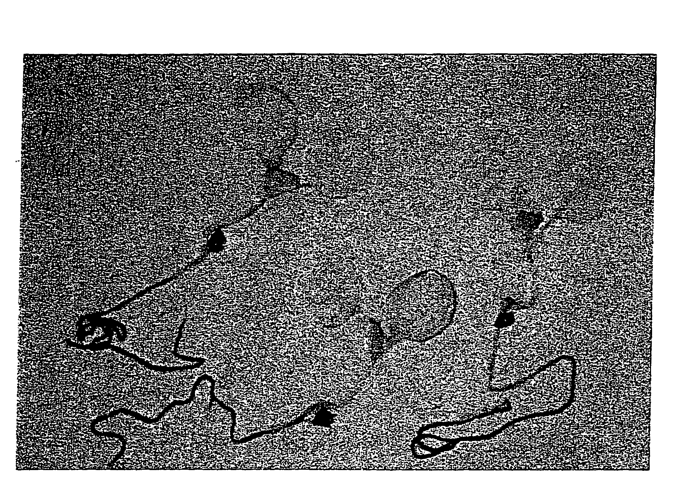

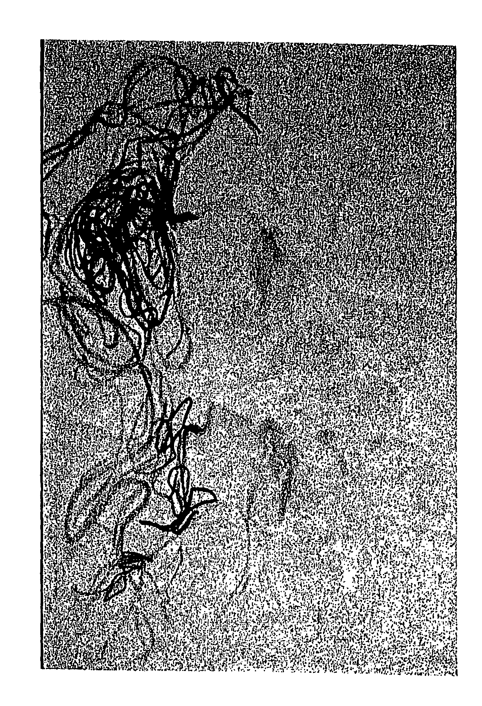

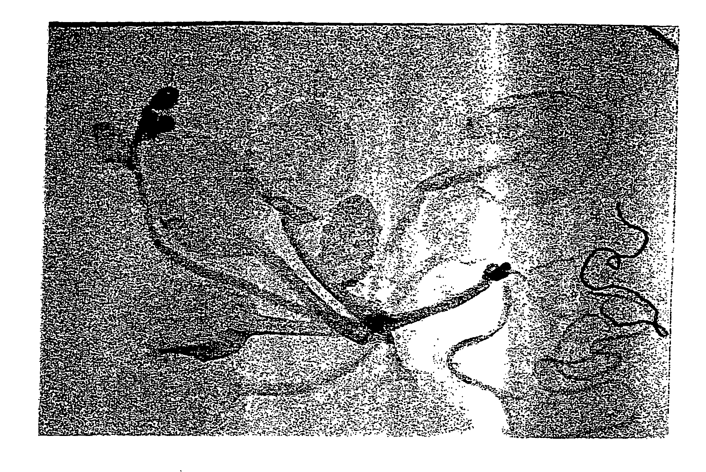

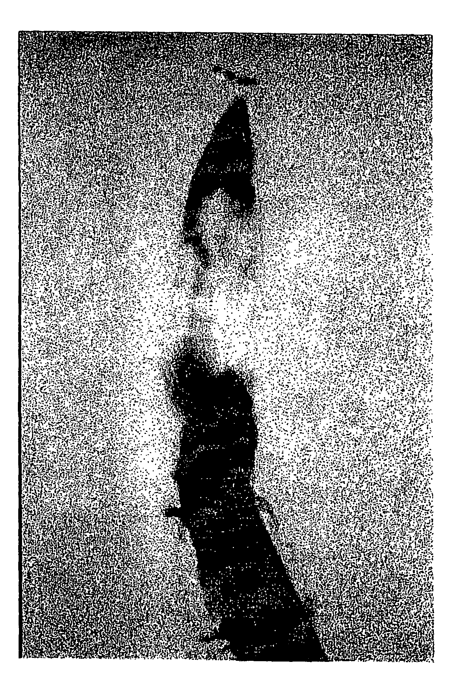

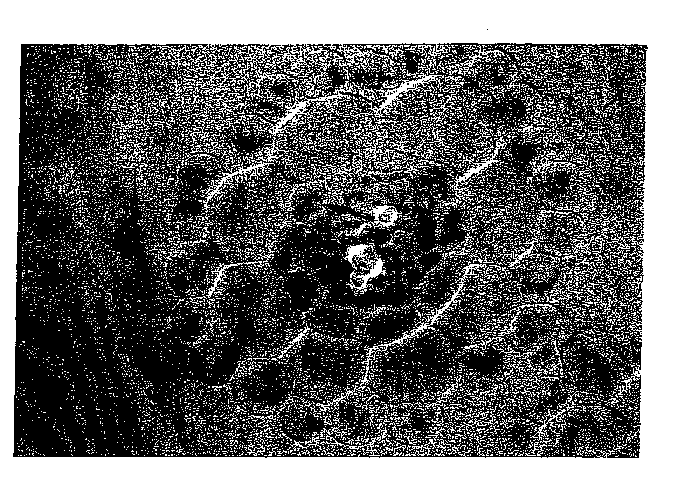

Un criblage de l'expression du gène GUS par histochimie parmi les transformants selon la méthode décrite par Mollier *et al*. (1995, C.R.A.S. Paris, volume 318: 465-474) a permis d'isoler un transformant présentant une activité GUS particulière:
- expression très forte spécifiquement dans la racine, et ce tout au long du développement, comme montré dans les clichés correspondant à la figure 3a-c. La racine est colorée sur toute la longueur sauf la zone d'élongation (figure 3d).
- expression dans toutes les assises cellulaires de la racine (épiderme, cortex, endoderme, péricycle, vaisseau conducteur) telle que cela peut être observé sur le cliché de la figure 4.

Ce transformant a été caractérisé plus avant par la technique de Southern blot (Southern E.M.,1975).

Une séquence d'environ 2,2 kb située en amont de la bordure droite de l'insertion, correspondant au promoteur, a été clonée par la technique de « Kanamycin Rescue » selon la technique décrite par Bouchez *et al*. (1996, Plant Mol. Biol. Rep. vol. 14:115-123).

Le vecteur de « Kanamycin rescue » est représenté en figure 7.

### EXEMPLE 2 : Recherche de la séquence complète du promoteur selon l'invention.

Le fragment d'ADN de 2,2 kb a été utilisé comme sonde pour rechercher le promoteur entier dans une banque d'ADN génomique d'*Arabidopsis thaliana* d'écotype Columbia (J.T. Mulligan, Stanford CA 943O5).

Deux phages d'environ 15 kb ont été sélectionnés (clones Ir1 et Ir2).. Ces deux clones de phages comportaient un insert correspondant à un fragment génomique de 4,413 kb (SEQ ID N°3) et contenant la séquence de la sonde. L'insert de ces deux phages a été entièrement séquencé par la méthode à l'exonucléase III décrite par Ausubel *et al*. (Current Protocols in Molecular Biology, éditeurs. F.M. AUSUBEL, R. BRENT., R.E. KINGSTON, D.D. MOORE, J.G. SEIDMAN, J.A. SMITH, K. STRUHL; published by Wiley Interscience). Il s'agit de la séquence SEQ ID N°3 . Le début de la séquence correspondant à l'ADN-T est localisé à partir du nucléotide en position 2285 de la séquence SEQ ID N°3.

L'expression spécifique du gène gus a été mise en évidence par des expériences de Northern blot sur des ARN totaux extraits de transformants homozygotes pour l'insertion.

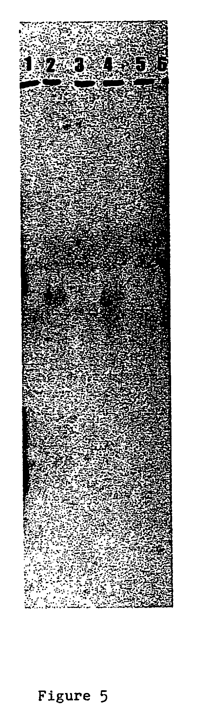

Les résultats d'une expérience de Northern blot sont représentés à la figure 5.

Un transcrit d'environ 2 kb est mis en évidence sur les ARN de racine et n'est pas détectable sur les ARN des parties aériennes (figure 5).

Les ARN totaux ont été extraits de racines et de parties aériennes de la lignée transformée selon la méthode décrite par Heim *et al*. (1993, Planta, vol. 191: 3.494, 3501).

En vue de mettre en évidence un éventuel transcrit endogène correspondant au promoteur, des gels de Northem ont été réalisés sur des ARN totaux extraits de plantes non transformées, et hybridées avec le fragment génomique de 4,413 kb (SEQ ID N°3) qui contient environ 2,2 kb en aval du promoteur.

Aucun transcrit n'a été détecté avec cette sonde.

En outre, deux banques d'ADNc d'*Arabidopsis thaliana* indépendantes (une banque d'ADNc de racines et une banque d'ADNc de plantes entières) ont été criblées avec cette même sonde de 4,413 kb.

A nouveau, les résultats ont été négatifs et aucun ADNc correspondant au promoteur n'a été trouvé.

Enfin, aucune phase codante n'a pu être mise en évidence en aval du promoteur en utilisant les logiciels classiques de prédiction.
Logiciels Net Plant Gene et Net Gene 2:
   S.M. Hebsgaard et al.(1996).
   Brunak S. et al. (1991).
Logiciel Genscan:
   Burge, C. et al. (1997);
   Burge, C.B. (1998).

La séquence de 4,413 kb (SEQ ID N°3) est très riche en bases A et T (68% de A et de T) et comporte 67 motifs ATG, 20 motifs CAAT, 38 motifs TATA, 9 motifs TATAAT et 2 boîtes Cr.

Les résultats obtenus indiquent qu'aucun transcrit n'est décelable en aval du promoteur étudié. Il s'agirait donc d'un promoteur cryptique.

### EXEMPLE 3: Mise en évidence de l'activité promotrice.

L'activité promotrice a été démontrée en réalisant une retransformation *in planta* d'*Arabidopsis thaliana* (écotype WS) par ce promoteur de 2,2 kb placé en amont du gène rapporteur gus.

Pour cette expérience, la construction suivante a été réalisée, qui est représentée sur la figure 2: un fragment d'environ 4,27 kb compris entre les sites Xbal et EcoRI du vecteur de « kanamycin Rescue » (cf. figure 7) a été cloné dans l'ADN-T du vecteur pBin19 selon la technique décrite par Bevan M. (1984, Nucleic Acid Research vo1.12: 8711-8721).

Ce fragment d'ADN de 4,27 kb est inclus dans la séquence SEQ ID N°4, cette séquence comprenant également un polysite de clonage du vecteur P38, comme décrit ci-après.

Il comporte: les sites de clonage de P38: Xbal, Spel, BamHI, Smal, Pstl (not 1 à 29), la séquence promotrice SEQ ID N°1 (nct 30 à 2178) et la séquence du gène *gus* de l'ADN-T de PGKB5 jusqu'au site EcoRI (nct 2179-4309).

### EXEMPLE 4: Transformation de plantes d'Arabidopsis thaliana avec la construction contenant le gène gus placé sous le contrôle du promoteur.

Des plantes d'*Arabidopsis thaliana* ont été transformées via *Agrobacterium tumefaciens* avec la construction 1 décrite dans la figure 2 et neuf transformants individuels ont été étudiés pour l'expression du gène *gus*, d'abord en histochimie.

L'expression du gène *gus* a également été quantifiée par dosage fluorimétrique selon la technique décrite par Jefferson (1987, Plant, Mol. Biol. Rep. volume 5: 387), modifiée par l'utilisation de 5mM de substrat dans les racines d'une part, et dans les parties aériennes (cotylédons, feuilles, tiges), d'autre part, et ce, à plusieurs stades du développement des plantes.

L'activité du gène *gus* des neuf transformants a été comparée à celle du transformant initial.

Les résultats sont représentés sur le tableau I ci-après.

Pour le transformant de départ ACC₆H, à l'état homozygote, ou ACC₆T₃, en ségrégation, l'activité du gène gus dans les racines diminue avec l'âge de la plante, alors qu'une faible activité dans les parties aériennes devient détectable en fin de développement.

Pour 6 des 9 transformants étudiés (transformants 6i, 6h, 6-1, 6-2, 6-3 et 6a) l'activité du gène gus dans les racines est encore plus forte que dans celle du transformant de départ (4 à dix fois ) et l'activité dans les feuilles devient plus facilement détectable.

Cependant, le rapport activité du gène *gus* dans les racines/activité du gène *gus* dans la feuille reste constant.

Pour ces transformants, la spécificité racinaire est donc inchangée par rapport au transformant de départ. Seul le niveau d'expression du gène *gus* est globalement plus élevé.

Pour 3 des transformants (6b, 2b et 6k) le rapport activité du gène *gus* dans la racine/activité du gène *gus* dans la feuille est plus faible que dans le transformant de départ; l'expression du gène *gus* est donc moins spécifique des racines pour ces transformants.

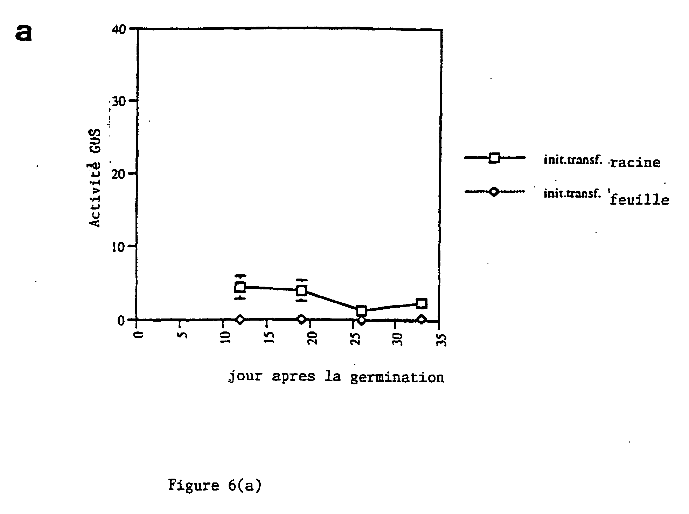

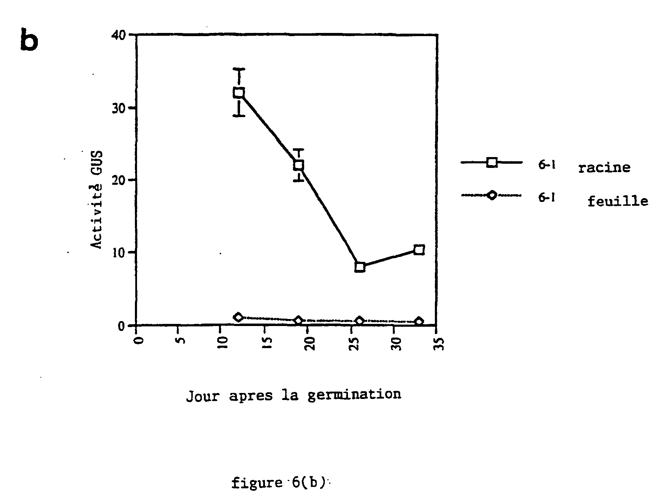

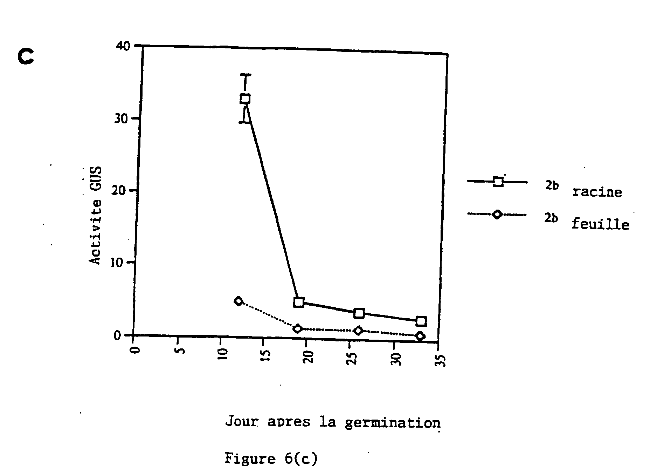

La diminution de l'activité GUS dans les racines au cours du développement est illustrée en figure 6, pour le transformant initial (a) et deux transformants caractéristiques (b et c). Pour le transformant initial, l'activité dans les feuilles n'est pas détectable. Pour le transformant 6-1, elle est faiblement détectable, et le rapport activité GUS racine/feuille est le même que pour le transformant initial. Pour le transformant 2b par contre, l'activité GUS dans les feuilles est plus élevée, et le rapport racine/feuille est nettement diminué.

### EXEMPLE 5 : Etude de délétions du promoteur selon l'invention

Des délétions du promoteur ont été obtenues selon la méthode à l'exonucléase III décrite dans la partie Matériels et Méthodes (Section IV) afin d'obtenir des fragments fonctionnels du promoteur.

### Digestion du fragment génomique à l'aide de l'exonucléase III

En premier lieu, le fragment génomique de 4.3 kb (sequence SEQ ID N° 3) a été cloné dans un vecteur pBluescript KS+, au site EcoRI, puis a subi des digestions partielles en 5' par l'exonuclease III.

### Clonage des fragments obtenus dans un vecteur d'expression fonctionnel chez les plantes

Afin de tester l'activité promotrice des fragments délétés du promoteur, les fragments obtenus après digestion enzymatique à l'aide de l'exonucléase III dans le vecteur pBluescript KS+, ont été amplifiés puis clonés dans un vecteur possédant le gène *gus*.

Les fragments du promoteur sont amplifiés par PCR à l'aide de 2 amorces portant des sites enzymatiques, respectivement:
- en 5' du promoteur, on utilise l'amorce T7-Hindlll, située dans le vecteur KS+ ;
- en 3' du promoteur, on utilise une amorce choisie dans la séquence génomique de 4.3kb et portant un site BamHI:

Il s'agit des amorces suivantes :
a) amorce T7-HindIII, en 5': GGC AAG CTT GTA ATA CGA CTC ACT ATA GGG C (SEQ ID N°6), qui possède la séquence "A/AGCTT" reconnue par l'endonucléase de restriction Hind III.
b) amorce en 3': CTA GGG ATC CAG CCA TTC CCT ATG C (SEQ ID N°7), qui possède la séquence "GGATC/C " reconnue par l'endonucléase de restriction BamHI. La séquence de cette amorce située du côté 5', par rapport au site BamHI est complémentaire de la séquence allant du nucléotide en position 2400 jusqu'au nucléotide en position 2386 de la séquence SEQ ID N°3.

### Protocole d'amplification par PCR

Sont mélangés pour chaque échantillon:
40 µl d'H₂O
5 µl tampon PCR 10x
1 µl dNTP 10 mM
1µl enzyme pfu-turbo DNA polymérase (à 2.5 u/µl, Stratagène)
1 µl d'amorce T7-Hind III ( à 10 mM)
1 µl d'amorce 4.4-BamHI ( à 10 mM)
1 µl d'ADN matrice (10 ng d'ADN du clone d'exonucléase choisi)

### Réaction PCR ::

L'amplification proprement dite est réalisée dans les conditions suivantes :
a) Etape de dénaturation pour l'obtention de fragments d'ADN simple brin à 94°C pendant 4 minutes ;
b) Trente cycles d'amplifications réalisés dans les conditions suivantes :
   - dénaturation à 94°C pendant 30 secondes ;
   - hybridation des amorces à 50°C pendant 45 secondes ;
   - élongation des amorces à 72°C pendant 3 minutes
c) Dernière étape d'élongation réalisée à 72°C pendant 10 minutes.

Les fragments de promoteur ainsi amplifiés comportent donc les sites Hind III, du côté 5', et BamHI, du côté 3'.

Les fragments amplifiés sont ensuite clonés au niveau des sites Hind III et BamHI, donc de façon orientée, dans le vecteur pC-*gus* dont la carte détaillée est représentée à la Figure 10. Le clonage a été réalisé conformément à la technique décrite dans la partie Matériels et Méthodes (Section III) pour le vecteur pBin19.

Les fragments clonés en amont du gène *gus* dans le vecteur pC-gus sont: les suivants :
- Le fragment allant du nucléotide en position 1 jusqu'au nucléotide en position 2400 de la séquence SEQ ID N°3 ;
Le fragment allant du nucléotide en position 493 jusqu'au nucléotide en position 2400 de la séquence SEQ ID N°3 ;
Le fragment allant du nucléotide en position 1076 jusqu'au nucléotide en position 2400 de la séquence SEQ ID N°3 ;
Le fragment allant du nucléotide en position 1976 jusqu'au nucléotide en position 2400 de la séquence SEQ ID N°3 ;
Le fragment allant du nucléotide en position 2040 jusqu'au nucléotide en position 2400 de la séquence SEQ ID N°3 ;

### Transformation de cellules d'Agrobacterium tumefaciens avec les vecteurs recombinants contenant divers fragments du promoteur selon l'invention

Les vecteurs pC-*gus* contenant les différents inserts sont ensuite transférés dans la souche d'Agrobactérie et des plantes d'*Arabidopsis* WS sont transformées conformément au protocole décrit à la Section I de la partie Matériels et Méthodes.

Les graines des transformants primaires sont sélectionnées sur un milieu sélectif contenant de l'hygromycine (30 mg/l).

La descendance de 20 transformants primaires par construction est semée sur milieu hygromycine afin de sélectionner les transformants possédant un seul locus d'insertion de l'ADN-T de *Agrobacterium tumefaciens*. Les homozygotes de ces transformants sont étudiés pour l'expression de la protéine GUS dans les racines et dans les feuilles, à la fois qualitativement par histochimie et quantitativement par fluorimétrie, conformément aux protocoles décrits dans la Section VI de la partie Materiels et Méthodes.

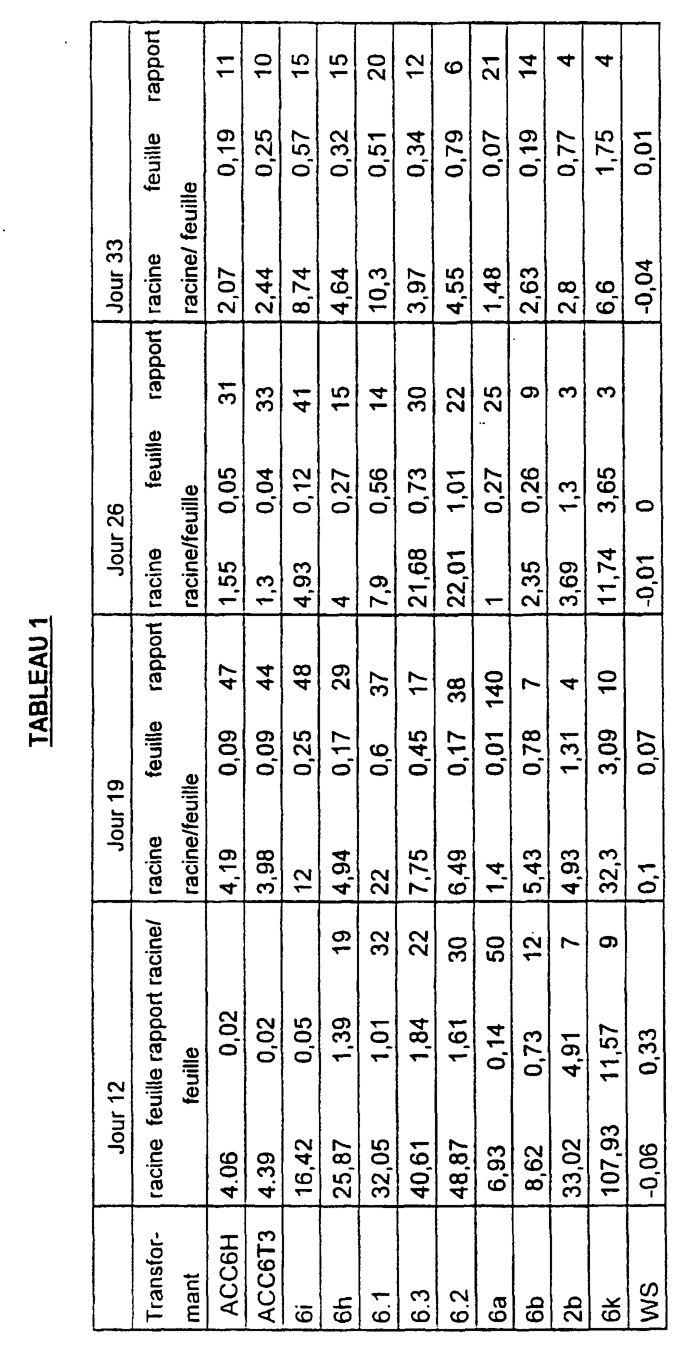

### REFERENCES BIBLIOGRAPHIQUES:

- F.M. AUSUBEL, R. BRENT, R.E. KINGSTON, D.D. MOORE, J.G. SEIDMAN, J.A. SMITH, K. STRUHL; 1989, Current Protocols in Molecular Biology published by Wiley Interscience.
- BASTOLA, D.R., PETHE, V.V. and WINICOV, I. (1998). Alfin 1 , a novel zinc finger protein in alfalfa roots that binds to promoter elements in the salt inducible MSPRP2 gène, Plant. Mol. Biol. 38, 1123-1135).
- BECHTOLD N. , Ellis J. PELLETIER G., 1993. *Agrobacterium* mediated gene transfer by infiltration of adult *Arabidopsis thaliana* plants. C.R. Acad. Sci. Paris 316: 1194-1199.
- BOUCHEZ D., CAMILLERI C., CABOCHE M. 1993. A binary vector based on Basta resistance for *in planta* transformation of *Arabidopsis thaliana*. C.R. Acad. Sci. Paris 316: 1188-1193.
- BEVAN M. 1984. Binary *Agrobacterium* vectors for plant transformation. Nucleic Acid Research 12: 8711-21.
- BRUNAK S. ENGELBRECHT, J. and KNUDSEN, S.: Prédiction of human mRNA Donor and Acceptor Sites from the DNA sequence. Journal of Molecular Biology, (1991), 220, 49-65.
- BURGE, C. and KARLIN, S. (1997). Prédiction of complete gene structures in human genomic DNA. J. Mol. Biol. 268, 78-94, BURGE, C.B. (1998). Modeling dependencies in pre-mRNA splicing signais. In SALZBERG, S. SEARLS, D. and KASIF; S. eds. Computational methods in molecular biology. Elsevier Science, Amsterdam, pp. 127-163.
- BOUCHEZ D. , VITTORIOSO P., COURTIAL B. CAMILLERI C., 1996. Kanamycin Rescue: A simple technique for the recovery of T-DNA flanking sequences. Plant Mol. Biol. Rep. 14: 115-123.
- BOUCHEZ D. CAMILLERI C., CABOCHE M. , 1993. A binary vector based on Basta résistance for *in planta* transformation of *Arabidopsis thaliana*. C.R. Acad. Sci. Paris 316: 1188-1193.
- CRAWFORD, N.M. and GLASS, A.D.M. 1998. Molecular and physiological aspects of nitrate uptake in plants. Trends in Plant Science, 3, 389-395.
- CORMACK , B.P. VALDIVIA, R.H. FALKOW, S. (1996), FACS-optimized mutants of the freen fluorescent protein (GFP) Gene 173: 33-38.
- JEFFERSON R. A., 1987. Assaying chimeric gènes in plants: the Gus gène fusion system. Plant Mol. Biol. Rep.5: 387.
- HEBSGAARD S.M., P.G. KORMING, N. TOLSTRUP, J. ENGELBRECHT, P. ROUZE, S. BRUNAK: Splice site prédiction in *Arabidopsis thaliana* DNA by combining local and global sequence information. Nucleic Acids Research, (1996), Vol.24, N°17, 3439-3452.
- HEIM U. WEBER H., BAUMLEIN H. WOBUS U. 1993. A sucrose-synthase gene of *Vicia faba L.* Expression pattern in developing seeds in relation to starch synthesis and metabolic régulation. *Planta* 191: 3494-501.
- HESSE, H. SONNEWALD, K. and WILLNITZER, L. (1995). Cloning and expression analysis of sucrose-phosphate synthase from sugar beet (*Beta vulgaris* L.) Mol. Gen. Genet, 247, 515-520).
- HWANG et al., (1995). An *Arabidopsis thaliana* root specific kinase homolog is induced by dehydration, ABA, and NaCl. Plant J.8: 37-43.
- HWANG I., GOODMAN, H.M. (1995). An *Arabidopsis thaliana* root specific kinase homolog is induced by deshydration, ABA, and NaCl, The Plant Journal, 8, 37-43).
- LEAH, R. TOMMERUP, H. SVENDSEN, I. and MUNDY, Y. (1991). Biochemical and molecular characterization of three barley seed proteins with antifungal properties . J. Biol. Chem. 266, 1464-1573).
- MALAMY, Y.E., BENFEY, P.N. (1997). Analysis of *scarecrow* expression using a rapid system for assessing transgene expression in *Arabidopsis* roots. Plant Y. 12: 957-963.
- MOLLIER P., MONTORO P., DELARUE M. BECHTOLD N. BELLINI C., PELLETIER G., 1995. Promoterless gus A expression in a large number of *Arabidopsis thaliana* transformants obtained by *in planta* infiltration method. C.R. Acd. Sci. Paris 318: 465-474.
- SOUTHERN , E.M. (1975) Detection of specific sequences among DNA fragments separated by gel electrophoresis. J. Mol. Biol. 98: 503-577.
- WINICOV, DEUTCH, C.E. (1994). Characterization of a CDNA clone from salt tolerant alfalfa cells that identifies salt-inducible root specific transcripts) J. Plant. Physiol. 144, 222-228).
- YANG T.T. CHENG, L. KAIN, S.R. (1996) Optimized codon usage and chromophore mutations provide enhanced sensitivity with the green fluorescent protein. Nucleic Acids Res. 24: 4592-4593.

## Revendications

1. Acide nucléique isolé ou purifié comprenant un promoteur végétal dirigeant spécifiquement l'expression d'une séquence nucléotidique d'intérêt dans les cellules de la racine d'une plante, durant la totalité du développement de cette dernière **caractérisé en ce que** ledit promoteur consiste en un polynucléotide possédant au moins 80 % d'identité en nucléotides avec un fragment d'au moins 400 nucléotides consécutifs de la séquence nucléotidique SEQ ID N°1, ou un acide nucléique de séquence complémentaire, à l'exception de la séquence référencée sous le N°AC 007 289 dans la base de données EMBL.

2. Acide nucléique selon la revendication 1, **caractérisé en ce qu'**il comprend un polynucléotide hybridant, dans des conditions d'hybridation de forte stringence, avec la séquence nucléotidique SEQ ID N°1, ou un acide nucléique de séquence complémentaire.

3. Acide nucléique selon l'une des revendications 1 ou 2, **caractérisé en ce que** ledit promoteur consiste en l'une des séquences suivantes:
- le polynucléotide allant du nucléotide en position 1 jusqu'au nucléotide en position 2400 de la séquence SEQ ID N°3;
- le polynucléotide allant du nucléotide en position 493 jusqu'au nucléotide en position 2400 de la séquence SEQ ID N°3;
- le polynucléotide allant du nucléotide en position 1076 jusqu'au nucléotide en position 2400 de la séquence SEQ ID N°3;
- le polynucléotide allant du nucléotide en position 1976 jusqu'au nucléotide en position 2400 de la séquence SEQ ID N°3; et
- le polynucléotide allant du nucléotide en position 2040 jusqu'au nucléotide en position 2400 de la séquence SEQ ID N°3.

4. Acide nucléique selon l'une des revendications 1 à 3, **caractérisé en ce qu'**il comprend une séquence nucléotidique d'intérêt placée sous le contrôle du promoteur végétal.

5. Acide nucléique selon la revendication 4, **caractérisé en ce qu'**il s'agit de la séquence nucléotidique SEQ ID N° 2.

6. Acide nucléique selon la revendication 4, **caractérisé en ce que** la séquence nucléotidique d'intérêt est choisie parmi les séquences codantes de gènes interagissant avec des parasites ou des pathogènes, les séquences codant les endochltinases, les séquences codant pour des protéines de protection de la plante au stress hydrique ou salin, ou encore des gènes agissant sur la teneur en sucre de la plante ou sur le transport de nitrate.

7. Acide nucléique comprenant 200 à 2000 nucléotides consécutifs d'un acide nucléique selon l'une des revendications 1 à 4, utile comme sonde ou amorce nucléotidique.

8. Vecteur recombinant de clonage et/ou d'expression comprenant un acide nucléique selon l'une des revendications 1 à 7.

9. Vecteur recombinant selon la revendication 8 , **caractérisé en ce qu'**il s'agit du vecteur contenu dans la souche *d'E. Coli* déposée à la CNCM le 25 Mai 1999 sous le Numéro d'accès I-2218.

10. Cellule hôte recombinante, **caractérisée en ce qu'**elle est transformée avec un adde nucléique selon l'une des revendications 1 à 7 ou un vecteur recombinant selon l'une des revendications 8 et 9.

11. Cellule hôte recombinante selon la revendication 10, **caractérisée en ce qu'**elle est d'origine bactérienne ou végétale.

12. Cellule hôte recombinante selon la revendication 11, **caractérisée en ce qu'**il s'agit d'une cellule d'*Agrobacterium tumefaciens*.

13. Cellule hôte recombinante selon l'une des revendications 10 ou 11, **caractérisée en ce qu'**il s'agit d'une cellule de la souche *d'E. Coli* déposée à la CNCM le 25 Mai 1999 sous le Numéro d'accès I-2218.

14. Organisme multicellulaire végétal recombinant, **caractérisé en ce qu'**il comprend une cellule hôte recombinante selon l'une des revendications 10 à 12.

15. Plante transgénique transformée avec un acide nuclêique selon l'une des revendications 1 à 7, ledit acide nucléique étant sous une forme intégrée dans le génome de ladite plante transgénique.

16. Plante transgénique selon la revendication 15, **caractérisée en ce qu'**il s'agit d'un colza, d'un tabac ou d'un maïs.

17. Procédé d'obtention d'une plante transgénique **caractérisé en ce qu'**il comprend les étapes suivantes :
a) Obtention d'une cellule hôte recombinante végétale selon l'une des revendications 10 ou 11 ;
b) Régénération d'une plante entière à partir de la cellule hôte recombinante obtenue à retape a).
c) Sélection des plantes obtenues à l'étape b) ayant intégré la séquence nucléotidique d'intérêt placée sous le contrôle du polynucléotide promoteur végétal.

18. Procédé d'obtention d'une plante transgénique **caractérisé en ce qu'**il comprend les étapes suivantes:
a) Obtention d'une cellule hôte recombinante d'*Agrobacterium tumefaciens* selon la revendication 12 ;
b) Transformation de la plante d'intérêt par infection avec la cellule hôte recombinante obtenue à l'étape a).
c) Sélection des plantes ayant intégré la séquence nucléotidique d'intérêt placée sous le contrôle du polynucléotide promoteur végétal.

19. Procédé d'obtention d'une plante transgénique **caractérisé en ce qu'**il comporte les étapes suivantes :
a) transfecter une cellule de plante avec un acide nucléique selon l'une des revendications 1 à 7 ou un vecteur recombinant selon l'une des revendications 8 ou 9;
b) régénération d'une plante entière à partir des cellules de plantes recombinantes obtenues à l'étape a) ;
c) sélection des plantes ayant intégré la séquence nucléotidique d'intérêt placée sous le contrôle du polynucléotide promoteur végétal.

20. Procédé d'obtention d'une plante transgénique selon l'une des revendications 17 à 19, **caractérisé en ce qu'**il comporte en outre les étapes de :
d) croisement entre elles de deux plantes transgéniques telles qu'obtenues à l'étape c) ;
e) sélection des plantes homozygotes pour le transgène.

21. Procédé d'obtention d'une plante transgénique selon l'une des revendications 17 à 19, **caractérisé en ce qu'**il comporte en outre les étapes de:
d) croisement d'une plante transgénique obtenue à l'étape c) avec une plante de la même espèce ;
e) sélection des plantes issues du croisement de l'étape d) ayant conservé le transgène.

22. Plante transformée avec un adde nucléique selon l'une des revendications 1 à 7 telle qu'obtenue selon le procédé selon l'une des revendications 17 à 21, et exprimant ledit acide nucléique spécifiquement au niveau des différents types cellulaires de la racine, à tous les stades de développement de la plante.

23. Semence d'une plante transgénique selon l'une des revendications 15, 16 et 22, ladite semence comprenant un acide nucléique selon l'une des revendications 1 à 7 sous une forme intégrée dans le génome des cellules qui la constituent.

## Patentansprüche

1. Isolierte oder gereinigte Nucleinsäure, umfassend einen pflanzlichen Promoter, der die Expression einer Nucleotidsequenz von Interesse in den Zellen der Wurzel einer Pflanze während der Gesamtheit der Entwicklung von Letzterer spezifisch steuert, **dadurch gekennzeichnet, dass** der Promoter aus einem Polynucleotid besteht, das, bezogen auf die Nucleotide, mindestens 80 % der Identität eines Fragments mit mindestens 400 aufeinander folgenden Nucleotiden der Nucleotidsequenz SEQ ID Nr. 1 oder einer Nucleinsäure mit einer komplementären Sequenz mit Ausnahme der in der Datenbank EMBL unter der Nr. AC 007 289 aufgeführten Sequenz aufweist.

2. Nucleinsäure nach Anspruch 1, **dadurch gekennzeichnet, dass** sie unter Hybridisierungsbedingungen mit starker Stringenz ein hybridisierendes Polynucleotid mit der Nucleotidsequenz SEQ ID Nr. 1 oder eine Nucleinsäure mit einer komplementären Sequenz umfasst.

3. Nucleinsäure nach einem der Ansprüche 1 oder 2, **dadurch gekennzeichnet, dass** der Promoter aus einer der folgenden Sequenzen besteht:
- dem Polynucleotid, das vom Nucleotid an Position 1 bis zum Nucleotid an Position 2400 von SEQ ID Nr. 3 reicht,
- dem Polynucleotid, das vom Nucleotid an Position 493 bis zum Nucleotid an Position 2400 von SEQ ID Nr. 3 reicht,
- dem Polynucleotid, das vom Nucleotid an Position 1076 bis zum Nucleotid an Position 2400 von SEQ ID Nr. 3 reicht,
- dem Polynucleotid, das vom Nucleotid an Position 1976 bis zum Nucleotid an Position 2400 von SEQ ID Nr. 3 reicht und
- dem Polynucleotid, das vom Nucleotid an Position 2040 bis zum Nucleotid an Position 2400 von SEQ ID Nr. 3 reicht.

4. Nucleinsäure nach einem der Ansprüche 1 bis 3, **dadurch gekennzeichnet, dass** sie eine Nucleotidsequenz von Interesse umfasst, die vom pflanzlichen Promoter gesteuert wird.

5. Nucleinsäure nach Anspruch 4, **dadurch gekennzeichnet, dass** es sich um die Nucleotidsequenz SEQ ID Nr. 2 handelt.

6. Nucleinsäure nach Anspruch 4, **dadurch gekennzeichnet, dass** die Nucleotidsequenz von Interesse aus den Sequenzen ausgewählt ist, die Gene, die mit Parasiten oder Krankheitserregern in Wechselwirkung stehen, Sequenzen, die die Endochitinasen kodieren, die Sequenzen, die für Proteine zum Schutz der Pflanze vor Wasser- oder Salzstress kodierend sind, oder darüber hinaus Gene, die den Zuckergehalt der Pflanze oder den Nitrattransport beeinflussen, kodieren.

7. Nucleinsäure, umfassend 200 bis 2000 aufeinander folgende Nucleotide einer Nucleinsäure nach einem der Ansprüche 1 bis 4, die als Nucleotidsonde oder -primer brauchbar sind.

8. Rekombinanter Klonierungs- und/oder Expressionsvektor, umfassend eine Nucleinsäure nach einem der Ansprüche 1 bis 7.

9. Rekombinanter Vektor nach Anspruch 8, **dadurch gekennzeichnet, dass** es sich um einen Vektor handelt, der in dem beim CNCM am 25. Mai 1999 unter der Hinterlegungsnummer 1-2218 hinterlegten Stamm von *E*. *Coli* enthalten ist.

10. Rekombinante Wirtszelle, **dadurch gekennzeichnet, dass** sie mit einer Nucleinsäure nach einem der Ansprüche 1 bis 7 oder einem rekombinanten Vektor nach einem der Ansprüche 8 und 9 transformiert ist.

11. Rekombinante Wirtszelle nach Anspruch 10, **dadurch gekennzeichnet, dass** sie einen bakteriellen oder pflanzlichen Ursprung hat.

12. Rekombinante Wirtszelle nach Anspruch 11, **dadurch gekennzeichnet, dass** es sich um eine Zelle von *Agrobacterium tumefaciens* handelt.

13. Rekombinante Wirtszelle nach einem der Ansprüche 10 oder 11, **dadurch gekennzeichnet, dass** es sich um eine Zelle des beim CNCM am 25. Mai 1999 unter der Hinterlegungsnummer 1-2218 hinterlegten Stamms von *E. Coli* handelt.

14. Rekombinanter, multizellulärer, pflanzlicher Organismus, **dadurch gekennzeichnet, dass** er eine rekombinante Wirtszelle nach einem der Ansprüche 10 bis 12 umfasst.

15. Transgene Pflanze, transformiert mit einer Nucleinsäure nach einem der Ansprüche 1 bis 7, wobei die Nucleinsäure in einer Form vorliegt, die in das Genom der transgenen Pflanze integriert ist.

16. Transgene Pflanze nach Anspruch 15, **dadurch gekennzeichnet, dass** es sich um Raps, Tabak oder Mais handelt.

17. Verfahren zur Herstellung einer transgenen Pflanze, **dadurch gekennzeichnet, dass** es die folgenden Schritte umfasst:
a) Herstellung einer pflanzlichen rekombinanten Wirtszelle nach einem der Ansprüche 10 oder 11,
b) Neubildung einer kompletten Pflanze ausgehend von der in Schritt a) erhaltenen rekombinanten Wirtszelle,
c) Auswahl von in Schritt b) erhaltenen Pflanzen, die die vom pflanzlichen Polynucleotid-Promoter gesteuerte Nucleotidsequenz von Interesse integriert enthalten.

18. Verfahren zur Herstellung einer transgenen Pflanze, **dadurch gekennzeichnet, dass** es die folgenden Schritte umfasst:
a) Herstellung einer rekombinanten Wirtszelle von *Agrobacterium tumefaciens* nach Anspruch 12,
b) Transformation der Pflanze von Interesse durch eine Infektion mit der in Schritt a) erhaltenen rekombinanten Wirtszelle,
c) Auswahl von Pflanzen, die die vom pflanzlichen Polynucleotid-Promoter gesteuerte Nucleotidsequenz von Interesse integriert enthalten.

19. Verfahren zur Herstellung einer transgenen Pflanze, **dadurch gekennzeichnet, dass** es die folgenden Schritte umfasst:
a) Transfizieren einer Pflanzenzelle mit einer Nucleinsäure nach einem der Ansprüche 1 bis 7 oder einem rekombinanten Vektor nach einem der Ansprüche 8 oder 9,
b) Neubildung einer kompletten Pflanze ausgehend von in Schritt a) erhaltenen Zellen von rekombinanten Pflanzen,
c) Auswahl von Pflanzen, die die vom pflanzlichen Polynucleotid-Promoter gesteuerte Nucleotidsequenz von Interesse integriert enthalten.

20. Verfahren zur Herstellung einer transgenen Pflanze nach einem der Ansprüche 17 bis 19, **dadurch gekennzeichnet, dass** es darüber hinaus die folgenden Schritte umfasst:
d) untereinander erfolgende Kreuzung von zwei transgenen Pflanzen wie denjenigen, die in Schritt c) erhalten wurden,
e) Auswahl von Pflanzen, die hinsichtlich des Transgens homozygot sind.

21. Verfahren zur Herstellung einer transgenen Pflanze nach einem der Ansprüche 17 bis 19, **dadurch gekennzeichnet, dass** es darüber hinaus die folgenden Schritte umfasst:
d) Kreuzung einer in Schritt c) erhaltenen transgenen Pflanze mit einer Pflanze derselben Art,
e) Auswahl von aus der Kreuzung von Schritt d) stammenden Pflanzen, in denen das Transgen erhalten ist.

22. Pflanze, transformiert mit einer Nucleinsäure nach einem der Ansprüche 1 bis 7 wie denjenigen, die nach dem Verfahren nach einem der Ansprüche 17 bis 21 erhalten wurden, und die die Nucleinsäure insbesondere im Bereich verschiedener Zelltypen der Wurzel in allen Entwicklungsstufen der Pflanze exprimiert.

23. Same einer transgenen Pflanze nach einem der Ansprüche 15, 16 und 22, wobei der Same eine Nucleinsäure nach einem der Ansprüche 1 bis 7 in einer Form umfasst, die in das Genom von Zellen, aus denen er besteht, integriert ist.

## Claims

1. Nucleic acid comprising all or part of a plant promoter capable of directing the expression of a nucleotide sequence of interest in the cells of the root of a plant throughout the entire development of this latter, **characterized in that** said promoter comprises all of part of a polynucleotide possessing at least 80% nucleotide identity with a fragment of at least 400 consecutive nucleotides of the nucleotide sequence SEQ ID No. 1 or a nucleic acid with the complementary sequence, with the exception of the sequence entered under the reference No. AC 007 289 in the EMBL data base.

2. Nucleic acid according to Claim 1, **characterized in that** it comprises polynucleotide hybridizing under hybridization conditions of high stringency with the nucleotide sequence SEQ ID No. 1 or a nucleic acid with the complementary sequence.

3. Nucleic acid according to one of the Claims 1 or 2, **characterized in that** it comprises of one of the following sequences:
- the polynucleotide extending from the nucleotide at position 1 to the nucleotide at position 2400 of the sequence SEQ ID No. 3;
- the polynucleotide extending from the nucleotide at position 493 to the nucleotide at position 2400 of the sequence SEQ ID No. 3;
- the polynucleotide extending from the nucleotide at position 1076 to the nucleotide at position 2400 of the sequence SEQ ID No. 3;
- the polynucleotide extending from the nucleotide at position 1976 to the nucleotide at position 2400 of the sequence SEQ ID No. 3; and
- the polynucleotide extending from the nucleotide at position 2040 to the nucleotide at position 2400 of the sequence SEQ ID No. 3.

4. Nucleic acid according to one of the Claims 1 to 3, **characterized in that** it comprises a nucleotide sequence of interest placed under the control of the plant promoter.

5. Nucleic acid according to Claim 4, **characterized in that** it is the nucleotide sequence SEQ ID No. 2.

6. Nucleic acid according to Claim 4, **characterized in that** the nucleotide sequence of interest is selected from the coding sequences of genes interacting with parasites or pathogens, the sequences coding for the endochitinases, the sequences coding for plant proteins protecting the plant from hydric or salt stress, or also genes acting on the sugar content of the plant or on nitrate transport.

7. Nucleic acid comprising 200 to 2000 consecutive nucleotides of a nucleic acid according to one of the Claims 1 to 4, useful as a nucleotide probe or primer.

8. Recombinant cloning and/or expression vector containing a nucleic acid according to one of the Claims 1 to 7.

9. Recombinant vector according to one of the Claims 8 or 9, **characterized in that** it is the vector contained in the *E. coli* strain deposited with the NCCM on 25 May 1999 under the access No. I-2218.

10. Recombinant cell host, **characterized in that** it is transformed by a nucleic acid according to one of the Claims 1 to 7 or a recombinant vector according to one of the Claims 8 and 9.

11. Recombinant host cell according to Claim 10, **characterized in that** it is of bacterial or plant origin.

12. Recombinant host cell according to Claim 11, **characterized in that** it is an *Agrobacterium tumefaciens* cell.

13. Recombinant host cell according to one of the Claims 10 or 11, **characterized in that** it is a cell of the *E. coli* strain deposited with the NCCM on 25 May 1999 under the access No. I-2218.

14. Recombinant plant multicellular organism, **characterized in that** it comprises a recombinant host cell according to one of the Claims 10 to 12.

15. Transgenic plant comprising in a form integrated in its genome a nucleic acid according to one of the Claims 1 to 7 said nucleic acid being in a form integrated in the genome of said transgenic plant.

16. Transgenic plant according to Claim 15, **characterized in that** it is colza, tobacco or maize.

17. Procedure for obtaining a transgenic plant **characterized in that** it comprises the following steps:
a) Production of a plant recombinant host cell according to one of the Claims 10 or 11;
b) Regeneration of a whole plant from the recombinant host cell obtained in step a).
c) Selection of the plants obtained in step b) which have integrated the nucleotide sequence of interest placed under the control of the plant polynucleotide promoter.

18. Procedure for producing a transgenic plant **characterized in that** it comprises the following steps:
a) Production of an *Agrobacterium tumefaciens* recombinant host cell according to Claim 12;
b) Transformation of the plant of interest by infection with the recombinant host cell obtained in step a).
c) Selection of the plants which have integrated the nucleotide sequence of interest placed under the control of the plant polynucleotide promoter.

19. Procedure for producing a transgenic plant **characterized in that** it comprises the following steps:
a) transfect a plant cell with a nucleic acid according to one of the Claims 1 to 7 or a recombinant vector according to either of the Claims 8 or 9;
b) regeneration of a whole plant from the recombinant host cells obtained in step a).
c) selection of the plants which have integrated the nucleotide sequence of interest placed under the control of the plant polynucleotide promoter.

20. Procedure for the production of a transgenic plant according to one of the Claims 17 to 19, **characterized in that** it comprises the additional steps:
d) cross of two transgenic plants such as obtained in step c);
e) selection of the plants homozygous for the transgene.

21. Procedure for the production of a transgenic plant according to one of the Claims 17 to 19, **characterized in that** it comprises the additional steps:
d) cross of a transgenic plant obtained in step c) with a plant of the same species;
e) selection of the plants derived from the cross of step d) which have conserved the transgene.

22. A transformed plant with a nucleic acid according to any of the claims 1 to 7, such as obtained according to the method of any of the claims 17 to 21, an expressing said nucleic acid specifically at the different cellular types of the root, at every step of the development of the plant.

23. A seed of a transgenic plant according to any of the claims 15, 16 and 22, said seed comprising a nucleic acid according to one of the claims 1 to 7 in a form integrated in the genome of the cells forming said seed.


### LISTE DE SEQUENCES

<110> Institut National de la Recherche Agronomique (INRA)
<120> Promoteur s'exprimant spécifiquement dans les cellules de racines de plantes, vecteurs et cellules hôtes recombinantes comprenant un tel promoteur et plantes transgéniques obtenues
<130> INRA Promoteur Végétal Racine
<140>
   <141>
<160> 7
<170> Patent In Ver. 2.1
<210> 1
   <211> 2149
   <212> ADN
   <213> Arabidopsis thaliana
<400> 1
   gtcgaattgt gatatattgt aagcaatctg aaaagaataa gtgggatata taaacaaccg 60
   gcgaaagtac aagttctacc tttttttggc atggaaccat gtttttagga tttactttgt 120
   aattcctgaa tctttcattt cttgaattga tatttacatt tttatcaaaa aaaaagtaca 180
   agttctacca aagcacagga gttaaacaac ttgtgtgtca aatgctaatt taaagcctaa 240
   tcttatgatt tccctttcct tcacgatata tactgatatt gatatgcacc catttgtttg 300
   tcattaactt cccactctat acatcagtat ctcaaagtcg aataacaata tccataagaa 360
   gtggtatatt gtgaaaaaaa aaaaaaaaag tggtatactg gtatatacaa taccacggtc 420
   tcgaattgcc tcaacaattt ctaggagaaa atggacgtgt ctctttggtt ttattttatt 480
   cttaataaca tactctatat tttaaacact tcgatgtctc gcttaaattt cgaatgtgcc 540
   taaatttctc taatcataaa tcgtaaagaa aattcgtcga agccacaggg acatgcatag 600
   ggcacgtagt tacctttaaa accatcaaaa atatattaat agaaaaggaa acttcctaaa 660
   agaacaattt aataaagtgg ataaaaaaag ataagaaggt aggcagaaga aaacgtatgg 720
   ccgcgactcg taacaaggga cgtcccgacc actgcggaga cggcgagacg ctgactgatt 780
   ttttcttttt cttttcctaa agaacgttgt ttcgtgctta caagggtcaa aaccatatcc 840
   aattgttctg cctattatta tataactaaa gatcccctct tgtgctttgt ctttattcgt 900
   gatatataat ctaacttaaa ttagttctaa aatatatatg tcctacctat gtttctactg 960
   acctcagtcc ctagttagct atatggacat atgtgaaaat gacgcccaaa atttgaagag 1020
   ttcctcttcc tgcaactaac tcttatctta ctcattgagc tatgttaaat attgaatgtt 1080
   ggcactctcg tattaaatat gccagttgca cctagataaa aaaacatgat agacatttag 1140
   tttaaaactt gaaatgttat ttgaactctt tggattacgt ggattgttgt atggattaaa 1200
   ttttgaagat atttatatat tgaagatgtt tatatatatt agagtttata tagcagaaaa 1260
   tattgatgta gatgttgtcc ttttgtagtt actctttttt gttgcgtagt cctttctcct 1320
   catcctccta tgaagaaaaa tccaaatagt ttaaggaaat ttttgtgtaa ttcatagtct 1380
   ttttcgtaac cacagttcta tgtagctatc gtcatcatat tcctctttgc aacaacaaaa 1440
   aagatcgttt ttgtaaaatt tagtagggca ctaaagtcgt catttgttgt cctgtcgaaa 1500
   tctagcgttc tgtcatccac aaataagttg tttgattcga gcttccaaga ttataatctt 1560
   ttttagatgg gtcatgaaga tttctaactt cgtatacgag tgtatccata taatttctaa 1620
   catatacgtc ttgtttttgg taggctctgc gtcttttgag accaccccct tgctaatgtt 1680
   ttgttgcacc ttagacaatc cataatacgt tacgtgagtc gaagttgcac caaaatggtc 1740
   caaatataat ttaaatttgg ccacaaaaca acattttaca aacaaattca acaaacatgc 1800
   atcgtttcaa attttattta ttcaatggcg ttatttgttc attgtaaata ttctgtttaa 1860
   ctcactgacg aattttttaa tttttcaaag aagaacattt ttgatataaa taacatttta 1920
   tggaaccacc ggttaagctc gatgattttg agttttagtt ttgtcgtttt gtgaaatcat 1980
   taacgaccta catttgatcc ctcattactt taataattag gaatcaaaca tgatgattaa 2040
   gttcaccaaa gacgtctctt atggctatta agagtcagac gcaaggatga ccggggtcat 2100
   taagacgtct tatattcaac cattactcca ctaattgcta attaatcag 2149
<210> 2
   <211> 4280
   <212> ADN
   <213> Séquence artificielle
<220>
   <223> Description de la séquence artificielle:Construction promoteur + séquence codante du gène gus
<400> 2
   gtcgaattgt gatatattgt aagcaatctg aaaagaataa gtgggatata taaacaaccg 60
   gcgaaagtac aagttctacc tttttttggc atggaaccat gtttttagga tttactttgt 120
   aattcctgaa tctttcattt cttgaattga tatttacatt tttatcaaaa aaaaagtaca 180
   agttctacca aagcacagga gttaaacaac ttgtgtgtca aatgctaatt taaagcctaa 240
   tcttatgatt tcccttttct tcacgatata tactgatatt gatatgcacc catttgtttg 300
   tcattaactt cccactctat acatcagtat ctcaaagtcg aataacaata tccataagaa 360
   gtggtatatt gtgaaaaaaa aaaaaaaaag tggtatactg gtatatacaa taccacggtc 420
   tcgaattgcc tcaacaattt ctaggagaaa atggacgtgt ctctttggtt ttattttatt 480
   cttaataaca tactctatat tttaaacact tcgatgtctc gcttaaattt cgaatgtgcc 540
   taaatttctc taatcataaa tcgtaaagaa aattcgtcga agccacaggg acatgcatag 600
   ggcacgtagt tacctttaaa accatcaaaa atatattaat agaaaaggaa acttcctaaa 660
   agaacaattt aataaagtgg ataaaaaaag ataagaaggt aggcagaaga aaacgtatgg 720
   ccgcgactcg taacaaggga cgtcccgacc actgcggaga cggcgagacg ctgactgatt 780
   ttttcttttt cttttcctaa agaacgttgt ttcgtgctta caagggtcaa aaccatatcc 840
   aattgttctg cctattatta tataactaaa gatcccctct tgtgctttgt ctttattcgt 900
   gatatataat ctaacttaaa ttagttctaa aatatatatg tcctacctat gtttctactg 960
   acctcagtcc ctagttagct atatggacat atgtgaaaat gacgcccaaa atttgaagag 1020
   ttcctcttcc tgcaactaac tcttatctta ctcattgagc tatgttaaat attgaatgtt 1080
   ggcactctcg tattaaatat gccagttgca cctagataaa aaaacatgat agacatttag 1140
   tttaaaactt gaaatgttat ttgaactctt tggattacgt ggattgttgt atggattaaa 1200
   ttttgaagat atttatatat tgaagatgtt tatatatatt agagtttata tagcagaaaa 1260
   tattgatgta gatgttgtcc ttttgtagtt actctttttt gttgcgtagt cctttctcct 1320
   catcctccta tgaagaaaaa tccaaatagt ttaaggaaat ttttgtgtaa ttcatagtct 1380
   ttttcgtaac cacagttcta tgtagctatc gtcatcatat tcctctttgc aacaacaaaa 1440
   aagatcgttt ttgtaaaatt tagtagggca ctaaagtcgt catttgttgt cctgtcgaaa 1500
   tctagcgttc tgtcatccac aaataagttg tttgattcga gcttccaaga ttataatctt 1560
   ttttagatgg gtcatgaaga tttctaactt cgtatacgag tgtatccata taatttctaa 1620
   catatacgtc ttgtttttgg taggctctgc gtcttttgag accaccccct tgctaatgtt 1680
   ttgttgcacc ttagacaatc cataatacgt tacgtgagtc gaagttgcac caaaatggtc 1740
   caaatataat ttaaatttgg ccacaaaaca acattttaca aacaaattca acaaacatgc 1800
   atcgtttcaa attttattta ttcaatggcg ttatttgttc attgtaaata ttctgtttaa 1860
   ctcactgacg aattttttaa tttttcaaag aagaacattt ttgatataaa taacatttta 1920
   tggaaccacc ggttaagctc gatgattttg agttttagtt ttgtcgtttt gtgaaatcat 1980
   taacgaccta catttgatcc ctcattactt taataattag gaatcaaaca tgatgattaa 2040
   gttcaccaaa gacgtctctt atggctatta agagtcagac gcaaggatga ccggggtcat 2100
   taagacgtct tatattcaac cattactcca ctaattgcta attaatcagt cccttatgtt 2160
   acgtcctgta gaaaccccaa cccgtgaaat caaaaaactc gacggcctgt gggcattcag 2220
   tctggatcgc gaaaactgtg gaattgatca gcgttggtgg gaaagcgcgt tacaagaaag 2280
   ccgggcaatt gctgtgccag gcagttttaa cgatcagttc gccgatgcag atattcgtaa 2340
   ttatgcgggc aacgtctggt atcagcgcga agtctttata ccgaaaggtt gggcaggcca 2400
   gcgtatcgtg ctgcgtttcg atgcggtcac tcattacggc aaagtgtggg tcaataatca 2460
   ggaagtgatg gagcatcagg gcggctatac gccatttgaa gccgatgtca cgccgtatgt 2520
   tattgccggg aaaagtgtac gtatcaccgt ttgtgtgaac aacgaactga actggcagac 2580
   tatcccgccg ggaatggtga ttaccgacga aaacggcaag aaaaagcagt cttacttcca 2640
   tgatttcttt aactatgccg gaatccatcg cagcgtaatg ctctacacca cgccgaacac 2700
   ctgggtggac gatatcaccg tggtgacgca tgtcgcgcaa gactgtaacc acgcgtctgt 2760
   tgactggcag gtggtggcca atggtgatgt cagcgttgaa ctgcgtgatg cggatcaaca 2820
   ggtggttgca actggacaag gcactagcgg gactttgcaa gtggtgaatc cgcacctctg 2880
   gcaaccgggt gaaggttatc tctatgaact gtgcgtcaca gccaaaagcc agacagagtg 2940
   tgatatctac ccgcttcgcg tcggcatccg gtcagtggca gtgaagggcc aacagttcct 3000
   gattaaccac aaaccgttct actttactgg ctttggtcgt catgaagatg cggacttacg 3060
   tggcaaagga ttcgataacg tgctgatggt gcacgaccac gcattaatgg actggattgg 3120
   ggccaactcc taccgtacct cgcattaccc ttacgctgaa gagatgctcg actgggcaga 3180
   tgaacatggc atcgtggtga ttgatgaaac tgctgctgtc ggctttaacc tctctttagg 3240
   cattggtttc gaagcgggca acaagccgaa agaactgtac agcgaagagg cagtcaacgg 3300
   ggaaactcag caagcgcact tacaggcgat taaagagctg atagcgcgtg acaaaaacca 3360
   cccaagcgtg gtgatgtgga gtattgccaa cgaaccggat acccgtccgc aagtgcacgg 3420
   gaatatttcg ccactggcgg aagcaacgcg taaactcgac ccgacgcgtc cgatcacctg 3480
   cgtcaatgta atgttctgcg acgctcacac cgataccatc agcgatctct ttgatgtgct 3540
   gtgcctgaac cgttattacg gatggtatgt ccaaagcggc gatttggaaa cggcagagaa 3600
   ggtactggaa aaagaacttc tggcctggca ggagaaactg catcagccga ttatcatcac 3660
   cgaatacggc gtggatacgt tagccgggct gcactcaatg tacaccgaca tgtggagtga 3720
   agagtatcag tgtgcatggc tggatatgta tcaccgcgtc tttgatcgcg tcagcgccgt 3780
   cgtcggtgaa caggtatgga atttcgccga ttttgcgacc tcgcaaggca tattgcgcgt 3840
   tggcggtaac aagaaaggga tcttcactcg cgaccgcaaa ccgaagtcgg cggcttttct 3900
   gctgcaaaaa cgctggactg gcatgaactt cggtgaaaaa ccgcagcagg gaggcaaaca 3960
   atgaatcaac aactctcctg gcgcaccatc gtcggctaca gcctcggtgg ggaattgagc 4020
   tcgatcgttc aaacatttgg caataaagtt tcttaagatt gaatcctgtt gccggtcttg 4080
   cgatgattat catataattt ctgttgaatt acgttaagca tgtaataatt aacatgtaat 4140
   gcatgacgtt atttatgaga tgggttttta tgattagagt cccgcaatta tacatttaat 4200
   acgcgataga aaacaaaata tagcgcgcaa actaggataa attatcgcgc gcggtgtcat 4260
   ctatgttact agatcgaatt 4280
<210> 3
   <211> 4413
   <212> ADN
   <213> Arabidopsis thaliana
<400> 3
   aagatccaca gtgaataaat aataagaacg gattcggtga tattgcaact atataatgaa 60
   attgaatact ctgattcatc gctttgtatc aagatcgaat ctctaaaaac atatactcta 120
   taataaatat ctgcagtcga attgtgatat attgtaagca atctgaaaag aataagtggg 180
   atatataaac aaccggcgaa agtacaagtt ctaccttttt ttggcatgga accatgtttt 240
   taggatttac tttgtaattc ctgaatcttt catttcttga attgatattt acatttttat 300
   caaaaaaaaa gtacaagttc taccaaagca caggagttaa acaacttgtg tgtcaaatgc 360
   taatttaaag cctaatctta tgatttccct tttcttcacg atatatactg atattgatat 420
   gcacccattt gtttgtcatt aacttcccac tctatacatc agtatctcaa agtcgaataa 480
   caatatccat aagaagtggt atattgtgaa aaaaaaaaaa aaaagtggta tactggtata 540
   tacaatacca cggtctcgaa ttgcctcaac aatttctagg agaaaatgga cgtgtctctt 600
   tggttttatt ttattcttaa taacatactc tatattttaa acacttcgat gtctcgctta 660
   aatttcgaat gtgcctaaat ttctctaatc ataaatcgta aagaaaattc gtcgaagcca 720
   cagggacatg catagggcac gtagttacct ttaaaaccat caaaaatata ttaatagaaa 780
   aggaaacttc ctaaaagaac aatttaataa agtggataaa aaaagataag aaggtaggca 840
   gaagaaaacg tatggccgcg actcgtaaca agggacgtcc cgaccactgc ggagacggcg 900
   agacgctgac tgattttttc tttttctttt cctaaagaac gttgtttcgt gcttacaagg 960
   gtcaaaacca tatccaattg ttctgcctat tattatataa ctaaagatcc cctcttgtgc 1020
   tttgtcttta ttcgtgatat ataatctaac ttaaattagt tctaaaatat atatgtccta 1080
   cctatgtttc tactgacctc agtccctagt tagctatatg gacatatgtg aaaatgacgc 1140
   ccaaaatttg aagagttcct cttcctgcaa ctaactctta tcttactcat tgagctatgt 1200
   taaatattga atgttggcac tctcgtatta aatatgccag ttgcacctag ataaaaaaac 1260
   atgatagaca tttagtttaa aacttgaaat gttatttgaa ctctttggat tacgtggatt 1320
   gttgtatgga ttaaattttg aagatattta tatattgaag atgtttatat atattagagt 1380
   ttatatagca gaaaatattg atgtagatgt tgtccttttg tagttactct tttttgttgc 1440
   gtagtccttt ctcctcatcc tcctatgaag aaaaatccaa atagtttaag gaaatttttg 1500
   tgtaattcat agtctttttc gtaaccacag ttctatgtag ctatcgtcat catattcctc 1560
   tttgcaacaa caaaaaagat cgtttttgta aaatttagta gggcactaaa gtcgtcattt 1620
   gttgtcctgt cgaaatctag cgttctgtca tccacaaata agttgtttga ttcgagcttc 1680
   caagattata atctttttta gatgggtcat gaagatttct aacttcgtat acgagtgtat 1740
   ccatataatt tctaacatat acgtcttgtt tttggtaggc tctgcgtctt ttgagaccac 1800
   ccccttgcta atgttttgtt gcaccttaga caatccataa tacgttacgt gagtcgaagt 1860
   tgcaccaaaa tggtccaaat ataatttaaa tttggccaca aaacaacatt ttacaaacaa 1920
   attcaacaaa catgcatcgt ttcaaatttt atttattcaa tggcgttatt tgttcattgt 1980
   aaatattctg tttaactcac tgacgaattt tttaattttt caaagaagaa catttttgat 2040
   ataaataaca ttttatggaa ccaccggtta agctcgatga ttttgagttt tagttttgtc 2100
   gttttgtgaa atcattaacg acctacattt gatccctcat tactttaata attaggaatc 2160
   aaacatgatg attaagttca ccaaagacgt ctcttatggc tattaagagt cagacgcaag 2220
   gatgaccggg gtcattaaga cgtcttatat tcaaccatta ctccactaat tgctaattaa 2280
   tcagattaat ttgtttaata cgataatgta ttttgattaa gtagctctca gccaacaggc 2340
   aaaggataaa ttttggatta ttcaaagatt tgtgggcttc caaaagcata gggaatggct 2400
   ccaactacat tgggaaatat attatttaaa tctccattcc catttgccac agtcgttgga 2460
   gtttattttt tttttccaag tggaagaatc aattataatt gtcggaattt ctaaatccta 2520
   cttgttggaa aacaaaccca agcaaaatta gtttagaaat gtacggaaat ctactataga 2580
   attatactaa acatatcaat atagctttga ctttaaaatt aaaacaatta ttgtgggcaa 2640
   ttagttagat attttaccag gggaaataag cagcactgtt catgcactct cttcttaatt 2700
   actatacttt gaaagaactt atatgtcact gtattgccag ttgccactaa tatataaaca 2760
   acatttcact tgttgacatc gctgtaaatg aagtttggaa cgaccctctt aacaactaat 2820
   agggttaatt aaccactaaa tttttccaag ttgtcatttt gtcttaatgt gagacgtaat 2880
   atctaatacg tcggtctaac ctaagagttg gttccgatca caaatttttg agaagtacct 2940
   ttcaataaaa aatttgttgt tataatttca ccgtgttaag taaaaactta attaggagct 3000
   attttctatt tgatgtgaat ttgaaaatgt cttcataaaa atagtatgga aaagggaatg 3060
   taattaatat agaccacaga tacaaaaaga tgtcccgtgc ttaacacgtc tgagtcattg 3120
   tcgtaccctt ttgccaactt tttcaagttt ctttcgtgaa aatgactaca ctttttaaaa 3180
   taaattgaca gatgattgtt gcatgcatat aatattcgca aaatgccaaa ttctaccctt 3240
   aaccaaataa tgggtaatgg atataaaata gttaacataa acaaaaattt ggaaattttg 3300
   aaaaatagag agtaaattga ttttttttaa aaagtttgaa ttgaagtgaa aaaatatata 3360
   ataaaaataa taaacctgta gtttgatata tatagttaga tagctcaagt ttgagtaact 3420
   tgaagtcttg aattacttta tatgtttttc tcacagatta tattattttc gttctatccc 3480
   aagaattggg ataatattct ctatattcga ggcctctctc ttaagaagtc ggtgtataat 3540
   cttaagccct tacttgacac aaggcctcta ttaaaaagcc caataataat ttttcttttc 3600
   aaagcccaac acgtcaagag gagaaagaag tgcgtttgcg tttggattga aaacgtggcg 3660
   ggcggttgga aactgaatct taaacccttc actcacattc atcttcaccg tcaaatctct 3720
   gaaactgagc ttcgacgatg ttgggtttac gaagatctgc gacgaccttg ttcgacatca 3780
   gccagtctct gcttcgtaat gttacggtta gtatctgtct aacacttcag cgccacattt 3840
   gggttttacg attagggttt caatagatcg aatcgattca tctctcttgg tattagattt 3900
   catcaaattc aaatctatcg tcacctaaaa atctctcttt cggtatgtag tatttccggg 3960
   gttgtttcaa agtcgtctta ttgctctgtg attttggctt cttacccatt ttcaacagtg 4020
   ctatgtagaa gaaagaacaa atctttgaaa tcgaaaggtc taatgtatag ttcaatgtct 4080
   acattatgag attgccatga tattatagtc aaacgattct cctaaagcgt ttacttttgt 4140
   ggagcattct ttgttagttc tatgcaaata aagttctagg aatgataatt cttaaggaag 4200
   catctcaaat gttggctagt tcttgtctca ggttaaaaca atgttttgca atttgctttt 4260
   agttagaatt gttgacttgc ttagcttttt gactaactct gtctctgtga agcaaagttt 4320
   gatcaaaccc attaccttat ttgatttctc tctttagatt atacatcaat ttatgtattt 4380
   tctttgtcta cagtttcatg ggttacgagt cca 4413
<210> 4
   <211> 4309
   <212> ADN
   <213> Séquence artificielle
<220>
   <223> Description de la séquence artificielle:Insert du vecteur pBin19
<400> 4
   tctagaacta gtggatcccc cgggctgcag tcgaattgtg atatattgta agcaatctga 60
   aaagaataag tgggatatat aaacaaccgg cgaaagtaca agttctacct ttttttggca 120
   tggaaccatg tttttaggat ttactttgta attcctgaat ctttcatttc ttgaattgat 180
   atttacattt ttatcaaaaa aaaagtacaa gttctaccaa agcacaggag ttaaacaact 240
   tgtgtgtcaa atgctaattt aaagcctaat cttatgattt cccttttctt cacgatatat 300
   actgatattg atatgcaccc atttgtttgt cattaacttc ccactctata catcagtatc 360
   tcaaagtcga ataacaatat ccataagaag tggtatattg tgaaaaaaaa aaaaaaaagt 420
   ggtatactgg tatatacaat accacggtct cgaattgcct caacaatttc taggagaaaa 480
   tggacgtgtc tctttggttt tattttattc ttaataacat actctatatt ttaaacactt 540
   cgatgtctcg cttaaatttc gaatgtgcct aaatttctct aatcataaat cgtaaagaaa 600
   attcgtcgaa gccacaggga catgcatagg gcacgtagtt acctttaaaa ccatcaaaaa 660
   tatattaata gaaaaggaaa cttcctaaaa gaacaattta ataaagtgga taaaaaaaga 720
   taagaaggta ggcagaagaa aacgtatggc cgcgactcgt aacaagggac gtcccgacca 780
   ctgcggagac ggcgagacgc tgactgattt tttctttttc ttttcctaaa gaacgttgtt 840
   tcgtgcttac aagggtcaaa accatatcca attgttctgc ctattattat ataactaaag 900
   atcccctctt gtgctttgtc tttattcgtg atatataatc taacttaaat tagttctaaa 960
   atatatatgt cctacctatg tttctactga cctcagtccc tagttagcta tatggacata 1020
   tgtgaaaatg acgcccaaaa tttgaagagt tcctcttcct gcaactaact cttatcttac 1080
   tcattgagct atgttaaata ttgaatgttg gcactctcgt attaaatatg ccagttgcac 1140
   ctagataaaa aaacatgata gacatttagt ttaaaacttg aaatgttatt tgaactcttt 1200
   ggattacgtg gattgttgta tggattaaat tttgaagata tttatatatt gaagatgttt 1260
   atatatatta gagtttatat agcagaaaat attgatgtag atgttgtcct tttgtagtta 1320
   ctcttttttg ttgcgtagtc ctttctcctc atcctcctat gaagaaaaat ccaaatagtt 1380
   taaggaaatt tttgtgtaat tcatagtctt tttcgtaacc acagttctat gtagctatcg 1440
   tcatcatatt cctctttgca acaacaaaaa agatcgtttt tgtaaaattt agtagggcac 1500
   taaagtcgtc atttgttgtc ctgtcgaaat ctagcgttct gtcatccaca aataagttgt 1560
   ttgattcgag cttccaagat tataatcttt tttagatggg tcatgaagat ttctaacttc 1620
   gtatacgagt gtatccatat aatttctaac atatacgtct tgtttttggt aggctctgcg 1680
   tcttttgaga ccaccccctt gctaatgttt tgttgcacct tagacaatcc ataatacgtt 1740
   acgtgagtcg aagttgcacc aaaatggtcc aaatataatt taaatttggc cacaaaacaa 1800
   cattttacaa acaaattcaa caaacatgca tcgtttcaaa ttttatttat tcaatggcgt 1860
   tatttgttca ttgtaaatat tctgtttaac tcactgacga attttttaat ttttcaaaga 1920
   agaacatttt tgatataaat aacattttat ggaaccaccg gttaagctcg atgattttga 1980
   gttttagttt tgtcgttttg tgaaatcatt aacgacctac atttgatccc tcattacttt 2040
   aataattagg aatcaaacat gatgattaag ttcaccaaag acgtctctta tggctattaa 2100
   gagtcagacg caaggatgac cggggtcatt aagacgtctt atattcaacc attactccac 2160
   taattgctaa ttaatcagtc ccttatgtta cgtcctgtag aaaccccaac ccgtgaaatc 2220
   aaaaaactcg acggcctgtg ggcattcagt ctggatcgcg aaaactgtgg aattgatcag 2280
   cgttggtggg aaagcgcgtt acaagaaagc cgggcaattg ctgtgccagg cagttttaac 2340
   gatcagttcg ccgatgcaga tattcgtaat tatgcgggca acgtctggta tcagcgcgaa 2400
   gtctttatac cgaaaggttg ggcaggccag cgtatcgtgc tgcgtttcga tgcggtcact 2460
   cattacggca aagtgtgggt caataatcag gaagtgatgg agcatcaggg cggctatacg 2520
   ccatttgaag ccgatgtcac gccgtatgtt attgccggga aaagtgtacg tatcaccgtt 2580
   tgtgtgaaca acgaactgaa ctggcagact atcccgccgg gaatggtgat taccgacgaa 2640
   aacggcaaga aaaagcagtc ttacttccat gatttcttta actatgccgg aatccatcgc 2700
   agcgtaatgc tctacaccac gccgaacacc tgggtggacg atatcaccgt ggtgacgcat 2760
   gtcgcgcaag actgtaacca cgcgtctgtt gactggcagg tggtggccaa tggtgatgtc 2820
   agcgttgaac tgcgtgatgc ggatcaacag gtggttgcaa ctggacaagg cactagcggg 2880
   actttgcaag tggtgaatcc gcacctctgg caaccgggtg aaggttatct ctatgaactg 2940
   tgcgtcacag ccaaaagcca gacagagtgt gatatctacc cgcttcgcgt cggcatccgg 3000
   tcagtggcag tgaagggcca acagttcctg attaaccaca aaccgttcta ctttactggc 3060
   tttggtcgtc atgaagatgc ggacttacgt ggcaaaggat tcgataacgt gctgatggtg 3120
   cacgaccacg cattaatgga ctggattggg gccaactcct accgtacctc gcattaccct 3180
   tacgctgaag agatgctcga ctgggcagat gaacatggca tcgtggtgat tgatgaaact 3240
   gctgctgtcg gctttaacct ctctttaggc attggtttcg aagcgggcaa caagccgaaa 3300
   gaactgtaca gcgaagaggc agtcaacggg gaaactcagc aagcgcactt acaggcgatt 3360
   aaagagctga tagcgcgtga caaaaaccac ccaagcgtgg tgatgtggag tattgccaac 3420
   gaaccggata cccgtccgca agtgcacggg aatatttcgc cactggcgga agcaacgcgt 3480
   aaactcgacc cgacgcgtcc gatcacctgc gtcaatgtaa tgttctgcga cgctcacacc 3540
   gataccatca gcgatctctt tgatgtgctg tgcctgaacc gttattacgg atggtatgtc 3600
   caaagcggcg atttggaaac ggcagagaag gtactggaaa aagaacttct ggcctggcag 3660
   gagaaactgc atcagccgat tatcatcacc gaatacggcg tggatacgtt agccgggctg 3720
   cactcaatgt acaccgacat gtggagtgaa gagtatcagt gtgcatggct ggatatgtat 3780
   caccgcgtct ttgatcgcgt cagcgccgtc gtcggtgaac aggtatggaa tttcgccgat 3840
   tttgcgacct cgcaaggcat attgcgcgtt ggcggtaaca agaaagggat cttcactcgc 3900
   gaccgcaaac cgaagtcggc ggcttttctg ctgcaaaaac gctggactgg catgaacttc 3960
   ggtgaaaaac cgcagcaggg aggcaaacaa tgaatcaaca actctcctgg cgcaccatcg 4020
   tcggctacag cctcggtggg gaattgagct cgatcgttca aacatttggc aataaagttt 4080
   cttaagattg aatcctgttg ccggtcttgc gatgattatc atataatttc tgttgaatta 4140
   cgttaagcat gtaataatta acatgtaatg catgacgtta tttatgagat gggtttttat 4200
   gattagagtc ccgcaattat acatttaata cgcgatagaa aacaaaatat agcgcgcaaa 4260
   ctaggataaa ttatcgcgcg cggtgtcatc tatgttacta gatcgaatt            4309
<210> 5
   <211> 7599
   <212> ADN
   <213> Séquence artificielle
<220>
   <223> Description de la séquence artificielle: ADN-T de PGKB5
<400> 5
   ggaaacagct atgaccatga ttacgccaag ctcggaatta accctcacta aagggaacaa 60
   aagctggagc tccaccgcgg tggcggccgc tctagaggat ccccccacag acagctccgt 120
   agccctcgtt ctccttggag ttcttcggga aatggatctt tcgattcccg atgatgtctc 180
   tcttatctgc tttgacgacg ccgactggac atccgctata acgccgccat tgaccgtgat 240
   ttcgcaacct gtcagggatc tcgcgacggc tgccacagaa gacctgatcg cccgcttaaa 300
   gggcgagact tcagccccac ccaaggaaac tcttctcccg gcggttctca tagagcgcgg 360
   ttccgtaagc ggttcttcgc aaggtcgggg ttgcataccg aactcgcgaa acgtcggcga 420
   ctgagctccc gaggcgcgtt gacaagatgc cacgaaggga atggaagaca gccgatattg 480
   caattgtctt cgtggactgc tttcgggacg taaggcgcaa gccatcatca ccgccgtcct 540
   aaacaaacat acctccacac aaatttatct acctgaccac aagatatatc ctgtcacacg 600
   atttattaaa cgctgcactt ggggtggtca gtcccttatg ttacgtcctg tagaaacccc 660
   aacccgtgaa atcaaaaaac tcgacggcct gtgggcattc agtctggatc gcgaaaactg 720
   tggaattgat cagcgttggt gggaaagcgc gttacaagaa agccgggcaa ttgctgtgcc 780
   aggcagtttt aacgatcagt tcgccgatgc agatattcgt aattatgcgg gcaacgtctg 840
   gtatcagcgc gaagtcttta taccgaaagg ttgggcaggc cagcgtatcg tgctgcgttt 900
   cgatgcggtc actcattacg gcaaagtgtg ggtcaataat caggaagtga tggagcatca 960
   gggcggctat acgccatttg aagccgatgt cacgccgtat gttattgccg ggaaaagtgt 1020
   acgtatcacc gtttgtgtga acaacgaact gaactggcag actatcccgc cgggaatggt 1080
   gattaccgac gaaaacggca agaaaaagca gtcttacttc catgatttct ttaactatgc 1140
   cggaatccat cgcagcgtaa tgctctacac cacgccgaac acctgggtgg acgatatcac 1200
   cgtggtgacg catgtcgcgc aagactgtaa ccacgcgtct gttgactggc aggtggtggc 1260
   caatggtgat gtcagcgttg aactgcgtga tgcggatcaa caggtggttg caactggaca 1320
   aggcactagc gggactttgc aagtggtgaa tccgcacctc tggcaaccgg gtgaaggtta 1380
   tctctatgaa ctgtgcgtca cagccaaaag ccagacagag tgtgatatct acccgcttcg 1440
   cgtcggcatc cggtcagtgg cagtgaaggg ccaacagttc ctgattaacc acaaaccgtt 1500
   ctactttact ggctttggtc gtcatgaaga tgcggactta cgtggcaaag gattcgataa 1560
   cgtgctgatg gtgcacgacc acgcattaat ggactggatt ggggccaact cctaccgtac 1620
   ctcgcattac ccttacgctg aagagatgct cgactgggca gatgaacatg gcatcgtggt 1680
   gattgatgaa actgctgctg tcggctttaa cctctcttta ggcattggtt tcgaagcggg 1740
   caacaagccg aaagaactgt acagcgaaga ggcagtcaac ggggaaactc agcaagcgca 1800
   cttacaggcg attaaagagc tgatagcgcg tgacaaaaac cacccaagcg tggtgatgtg 1860
   gagtattgcc aacgaaccgg atacccgtcc gcaagtgcac gggaatattt cgccactggc 1920
   ggaagcaacg cgtaaactcg acccgacgcg tccgatcacc tgcgtcaatg taatgttctg 1980
   cgacgctcac accgatacca tcagcgatct ctttgatgtg ctgtgcctga accgttatta 2040
   cggatggtat gtccaaagcg gcgatttgga aacggcagag aaggtactgg aaaaagaact 2100
   tctggcctgg caggagaaac tgcatcagcc gattatcatc accgaatacg gcgtggatac 2160
   gttagccggg ctgcactcaa tgtacaccga catgtggagt gaagagtatc agtgtgcatg 2220
   gctggatatg tatcaccgcg tctttgatcg cgtcagcgcc gtcgtcggtg aacaggtatg 2280
   gaatttcgcc gattttgcga cctcgcaagg catattgcgc gttggcggta acaagaaagg 2340
   gatcttcact cgcgaccgca aaccgaagtc ggcggctttt ctgctgcaaa aacgctggac 2400
   tggcatgaac ttcggtgaaa aaccgcagca gggaggcaaa caatgaatca acaactctcc 2460
   tggcgcacca tcgtcggcta cagcctcggt ggggaattga gctcgatcgt tcaaacattt 2520
   ggcaataaag tttcttaaga ttgaatcctg ttgccggtct tgcgatgatt atcatataat 2580
   ttctgttgaa ttacgttaag catgtaataa ttaacatgta atgcatgacg ttatttatga 2640
   gatgggtttt tatgattaga gtcccgcaat tatacattta atacgcgata gaaaacaaaa 2700
   tatagcgcgc aaactaggat aaattatcgc gcgcggtgtc atctatgtta ctagatcgaa 2760
   ttcgatcgag gggatcgagc ccctgctgag cctcgacatg ttgtcgcaaa attcgccctg 2820
   gacccgccca acgatttgtc gtcactgtca aggtttgacc tgcacttcat ttggggccca 2880
   catacaccaa aaaaatgctg cataattctc ggggcagcaa gtcggttacc cggccgccgt 2940
   gctggaccgg gttgaatggt gcccgtaact ttcggtagag cggacggcca atactcaact 3000
   tcaaggaatc tcacccatgc gcgccggcgg ggaaccggag ttcccttcag tgaacgttat 3060
   tagttcgccg ctcggtgtgt cgtagatact agcccctggg gccttttgaa atttgaataa 3120
   gatttatgta atcagtcttt taggtttgac cggttctgcc gcttttttta aaattggatt 3180
   tgtaataata aaacgcaatt gtttgttatt gtggcgctct atcatagatg tcgctataaa 3240
   cctattcagc acaatatatt gttttcattt taatattgta catataagta gtagggtaca 3300
   atcagtaaat tgaacggaga atattattca taaaaatacg atagtaacgg gtgatatatt 3360
   cattagaatg aaccgaaacc ggcggtaagg atctgagcta cacatgctca ggttttttac 3420
   aacgtgcaca acagaattga aagcaaatat catgcgatca taggcgtctc gcatatctca 3480
   ttaaagcagg gggtgggcga agaactccag catgagatcc ccgcgctgga ggatcatcca 3540
   gccggcgtcc cggaaaacga ttccgaagcc caacctttca tagaaggcgg cggtggaatc 3600
   gaaatctcgt gatggcaggt tgggcgtcgc ttggtcggtc atttcgaacc ccagagtccc 3660
   gctcagaaga actcgtcaag aaggcgatag aaggcgatgc gctgcgaatc gggagcggcg 3720
   ataccgtaaa gcacgaggaa gcggtcagcc cattcgccgc caagctcttc agcaatatca 3780
   cgggtagcca acgctatgtc ctgatagcgg tccgccacac ccagccggcc acagtcgatg 3840
   aatccagaaa agcggccatt ttccaccatg atattcggca agcaggcatc gccatgggtc 3900
   acgacgagat cctcgccgtc gggcatgcgc gccttgagcc tggcgaacag ttcggctggc 3960
   gcgagcccct gatgctcttc gtccagatca tcctgatcga caagaccggc ttccatccga 4020
   gtacgtgctc gctcgatgcg atgtttcgct tggtggtcga atgggcaggt agccggatca 4080
   agcgtatgca gccgccgcat tgcatcagcc atgatggata ctttctcggc aggagcaagg 4140
   tgagatgaca ggagatcctg ccccggcact tcgcccaata gcagccagtc ccttcccgct 4200
   tcagtgacaa cgtcgagcac agctgcgcaa ggaacgcccg tcgtggccag ccacgatagc 4260
   cgcgctgcct cgtcctgcag ttcattcagg gcaccggaca ggtcggtctt gacaaaaaga 4320
   accgggcgcc cctgcgctga cagccggaac acggcggcat cagagcagcc gattgtctgt 4380
   tgtgcccagt catagccgaa tagcctctcc acccaagcgg ccggagaacc tgcgtgcaat 4440
   ccatcttgtt caatccacat gatcatgggc cggatctttg attgagagtg aatatgagac 4500
   tctaattgga taccgagggg aatttatgga acgtcagtgg agcatttttg acaagaaata 4560
   tttgctagct gatagtgacc ttaggcgact tttgaacgcg caataatggt ttctgacgta 4620
   tgtgcttagc tcattaaact ccagaaaccc gcggctgagt ggctccttca atcgttgcgg 4680
   ttctgtcagt tccaaacgta aaacggcttg tcccgcgtca tcggcggggg tcataacgtg 4740
   actcccttaa ttctccgctc atgatcctgt ttcctgtgtg aaattgttat ccgctcacaa 4800
   ttccacacat tatacgagcc ggaagcataa agtgtaaagc ctggggtgcc taatgagtga 4860
   gctaactcac attaattgcg ttgcgctcac tgcccgcttt ccagtcggga aacctgtcgt 4920
   gccagctgca ttaatgaatc ggaattgacg gatctccttt gccccggaga tcaccatgga 4980
   cgactttctc tatctctacg atctaggaag aaagttcgac ggagaaggtg acgataccat 5040
   gttcaccacc gataatgaga agattagcct cttcaatttc agaaagaatg ctgacccaca 5100
   gatggttaga gaggcctacg cggcaggtct catcaagacg atctacccga gtaataatct 5160
   ccaggagatc aaataccttc ccaagaaggt taaagatgca gtcaaaagat tcaggactaa 5220
   ctgcatcaag aacacagaga aagatatatt tctcaagatc agaagtacta ttccagtatg 5280
   gacgattcaa ggcttgcttc ataaaccaag gcaagtaata gagattggag tctctaagaa 5340
   agtagttcct actgaatcaa aggccatgga gtcaaaaatt cagatcgagg atctaacaga 5400
   actcgccgtg aagactggcg aacagttcat acagagtctt ttacgactca atgacaagaa 5460
   gaaaatcttc gtcaacatgg tggagcacga cactctcgtc tactccaaga atatcaaaga 5520
   tacagtctca gaagaccaaa gggctattga gacttttcaa caaagggtaa tatcgggaaa 5580
   cctcctcgga ttccattgcc cagctatctg tcacttcatc aaaaggacag tagaaaagga 5640
   aggtggcacc tacaaatgcc atcattgcga taaaggaaag gctatcgttc aagatgcctc 5700
   tgccgacagt ggtcccaaag atggaccccc acccacgagg agcatcgtgg aaaaagaaga 5760
   cgttccaacc acgtcttcaa agcaagtgga ttgatgtgat atctccactg acgtaaggga 5820
   tgacgcacaa tcccactatc cttcgcaaga cccttcctct atataaggaa gttcatttca 5880
   tttggagagg acacgctgaa atcaccagtc tctctctaca aatcggatcc atgagcccag 5940
   aacgacgccc ggccgacatc cgccgtgcca ccgaggcgga catgccggcg gtctgcacca 6000
   tcgtcaacca ctacatcgag acaagcacgg tcaacttccg taccgagccg caggaaccgc 6060
   aggagtggac ggacgacctc gtccgtctgc gggagcgcta tccctggctc gtcgccgagg 6120
   tggacggcga ggtcgccggc atcgcctacg cgggcccctg gaaggcacgc aacgcctacg 6180
   actggacggc cgagtcgacc gtgtacgtct ccccccgcca ccagcggacg ggactgggct 6240
   ccacgctcta cacccacctg ctgaagtccc tggaggcaca gggcttcaag agcgtggtcg 6300
   ctgtcatcgg gctgcccaac gacccgagcg tgcgcatgca cgaggcgctc ggatatgccc 6360
   cccgcggcat gctgcgggcg gccggcttca agcacgggaa ctggcatgac gtgggtttct 6420
   ggcagctgga cttcagcctg ccggtaccgc cccgtccggt cctgcccgtc accgagatct 6480
   gatctcacgc gtctaggatc cgatggatcc cccgatgagc taagctagct atatcatcaa 6540
   tttatgtatt acacataata tcgcactcag tctttcatct acggcaatgt accagctgat 6600
   ataatcagtt attgaaatat ttctgaattt aaacttgcat caataaattt atgtttttgc 6660
   ttggactata atacctgact tgttatttta tcaataaata tttaaactat atttctttca 6720
   agatgggaat taacatctac aaattgcctt ttcttatcga ccatgtacat caagcttatc 6780
   gataccgtcg gctattggta ataggacact gggattcgtc ttggacaact ttccttctca 6840
   tctaagcgta gacaaccctc aactggaaac gggccggact ccagggcgtg tgccaggtgc 6900
   ccacggaata gttttggcca gacccttgaa aatccgattc agtacaatcg attgccctca 6960
   tttttacgtt ggcatatatc ctgccaaaca gccaacaacg cgcgtgcggt gaataggaaa 7020
   gcgtttgagt tgcttgctca tatcgtgacg gttgacagca caggttgacc gcttgatgat 7080
   tcgtacgagc cgccaaacat tggctgtcgt aatgatatac catgtcagaa cagcaatccg 7140
   atggggcgga aagcattatc ttaatgcaca cggaaatggc gcgtcggtgg gtggaataca 7200
   ccgacataga ggccgtaagt tctgcatggt catcgtcgga aaggtggcag caggcgcacg 7260
   gctgtggcct cttgctcttt cagcgtgaaa tgcgtgttga aagaataatc gaagagagcg 7320
   tccgctcgac accttcaatt atgccgattt gatcgatgaa ctgatcgagc tctgaaatcg 7380
   aaggggcttc gataatcgca atcaaatcaa aagtgccact cacagaatga agagcgataa 7440
   cggccgtgac cttcccaagg gaggccgtca cctgtgaaag cgccttcgta atggtgatca 7500
   gaatatgggc tcgaaccaag ctcgagacct cgaggggggg cccggtaccc aattcgccct 7560
   atagtgagtc gtattacaat tcactggccg tcgttttac 7599
<210> 6
   <211> 31
   <212> ADN
   <213> Arabidopsis thaliana
<400> 6
   ggcaagcttg taatacgact cactataggg c 31
<210> 7
   <211> 25
   <212> ADN
   <213> Arabidopsis thaliana
<400> 7
   ctagggatcc agccattccc tatgc 25